# EUROPEAN PATENT APPLICATION

(11) **EP 4 473 985 A1**
(43) Date of publication of application: **11.12.2024**
(21) Application number: 24176886.0
(22) Date of filing: 20.05.2024
(51) Int. Cl.: A61M 1/16, A61M 1/26

(54) **OXYGENATOR WITH CIRCUMFERENTIALLY CONSTRICTING HOUSING**

(30) Priority: 01.06.2023 US 202318204739
(71) Applicant: CardiacAssist, Inc., Pittsburgh, PA 15238 (US)
(72) Inventor: HARTOGS, Ruben, Pittsburgh, PA 15232 (US); KELLEHER, Kelli, Glenshaw, PA 15116 (US); SVITEK, Robert, Freeport, PA 16229 (US); SIMPSON, Luke, Pittsburgh, PA 15211 (US)
(74) Representative: Buzzi, Notaro & Antonielli d'Oulx S.p.A.

(57) **Abstract**

An oxygenator may include a housing extending from a first end to a second end, and a bundle of hollow gas exchange fibers extending between the first end and the second end. At least a portion of the housing may be configured to selectively move radially with respect to the bundle of hollow gas exchange fibers. An oxygenator may include a rigid housing extending from a first end to a second end, a flexible housing disposed within the rigid housing, and a bundle of hollow gas exchange fibers extending between the first end and the second end within the flexible housing. At least a portion of the flexible housing may be configured to selectively move radially with respect to the bundle of hollow gas exchange fibers.

## Description

### TECHNICAL FIELD

The present disclosure relates generally to extracorporeal blood conditioning devices and methods for conditioning blood in extracorporeal circulation.

### BACKGROUND

Generally, hollow fiber blood oxygenators that are used to exchange oxygen (O2) and carbon dioxide (CO2) in extracorporeal circulation have one of two forms. Either the oxygenator has a cylindrical housing with a crisscross hollow fiber mat spirally wound around a cylindrical central core, or the oxygenator has a polygonal housing with stacked (piled) hollow fiber mat layers. Blood flows outside the hollow fiber lumens and oxygenating gases flow inside the hollow fiber lumens.

In cylindrically wound oxygenators, blood flow across the hollow fibers may be radial, longitudinal, circumferential, or a combination of two or more of these. In some cases, this may lead to a blood flow path that is relatively long and non-laminar to guarantee high gas exchange efficiency with reduced surface area and limited device priming volume, which are important parameters for optimal patient perfusion.

In some oxygenators, blood stagnation may occur in isolated areas where low (or lower) blood flow occurs. Blood tends to follow the path of least resistance through the oxygenator, while a uniform flow and/or resistance is desirable. Locations where blood flow is reduced may stagnate, resulting in thrombus formation. Alternative oxygenator configurations which may disrupt the blood flow path and/or enhance mixing of blood within the oxygenator may result in reduced stagnation, reduced thrombus formation, and/or improved oxygenation.

There is an ongoing need for alternative devices, components, and/or methods of use and/or manufacture of said devices and/or components.

### SUMMARY

In one example, an oxygenator may comprise a housing extending from a first end to a second end, and a bundle of hollow gas exchange fibers extending between the first end and the second end. At least a portion of the housing may be configured to selectively move radially with respect to the bundle of hollow gas exchange fibers.

In addition or alternatively to any example disclosed herein, the housing includes a flexible portion disposed between the first end and the second end.

In addition or alternatively to any example disclosed herein, the housing may further include a second flexible portion disposed between the first end and the second end.

In addition or alternatively to any example disclosed herein, the second flexible portion is configured to selectively move radially with respect to the bundle of hollow gas exchange fibers independently of the flexible portion.

In addition or alternatively to any example disclosed herein, a wall of the housing extending between the first end and the second end is configured to shift between radially inward and radially outward positions.

In addition or alternatively to any example disclosed herein, the housing includes an actuation apparatus configured to selectively move radially with respect to the bundle of hollow gas exchange fibers.

In addition or alternatively to any example disclosed herein, the actuation apparatus is configured to operate in a cyclical manner.

In addition or alternatively to any example disclosed herein, the actuation apparatus is configured to operate in a variable manner.

In addition or alternatively to any example disclosed herein, the actuation apparatus comprises a plurality of rings disposed between the first end and the second end.

In addition or alternatively to any example disclosed herein, the plurality of rings is configured to move radially with respect to the bundle of hollow gas exchange fibers sequentially along the bundle of hollow gas exchange fibers.

In addition or alternatively to any example disclosed herein, an oxygenator may comprise a rigid housing extending from a first end to a second end, a flexible housing disposed within the rigid housing, and a bundle of hollow gas exchange fibers extending between the first end and the second end within the flexible housing. At least a portion of the flexible housing may be configured to selectively move radially with respect to the bundle of hollow gas exchange fibers.

In addition or alternatively to any example disclosed herein, the flexible housing is disposed radially outward of the bundle of hollow gas exchange fibers and a fluid is disposed between the rigid housing and the flexible housing, wherein changes in pressure of the fluid cause at least a portion of the flexible housing to move radially with respect to the bundle of hollow gas exchange fibers.

In addition of alternatively to any example disclosed herein, the flexible housing is disposed within the bundle of hollow gas exchange fibers and a fluid is disposed within the flexible housing, wherein changes in pressure of the fluid cause at least a portion of the flexible housing to move radially with respect to the bundle of hollow gas exchange fibers.

In addition or alternatively to any example disclosed herein, the flexible housing includes an expandable bladder formed within a wall of the flexible housing.

In addition or alternatively to any example disclosed herein, the expandable bladder is configured to receive an inflation fluid therein such that changes in pressure or volume of the inflation fluid within the expandable bladder cause at least a portion of the wall of the flexible housing to move radially with respect to the bundle of hollow gas exchange fibers.

In addition or alternatively to any example disclosed herein, the expandable bladder is disposed at a position radially outward of the bundle of hollow gas exchange fibers.

In addition or alternatively to any example disclosed herein, the expandable bladder is disposed at a position radially inward of the bundle of hollow gas exchange fibers.

In addition or alternatively to any example disclosed herein, the oxygenator may further comprise an annular ring disposed in contact with the flexible housing.

In addition or alternatively to any example disclosed herein, the annular ring is movable axially along the flexible housing.

In addition or alternatively to any example disclosed herein, the annular ring is disposed around the bundle of hollow gas exchange fibers between the rigid housing and the flexible housing and has an axial length less than a length of the flexible housing. The flexible housing has a first diameter in an unconstrained configuration. The annular ring has an inner diameter less than the first diameter of the flexible housing such that the annular ring radially constricts a portion of the flexible housing disposed therein to a second diameter less than the first diameter.

In addition or alternatively to any example disclosed herein, the annular ring is disposed within the flexible housing and has an axial length less than a length of the flexible housing. The flexible housing has a first diameter in an unconstrained configuration. The annular ring has an outer diameter greater than the first diameter of the flexible housing such that the annular ring radially expands a portion of the flexible housing to a second diameter greater than the first diameter

In addition or alternatively to any example disclosed herein, an oxygenator may comprise a housing extending from a first end to a second end, and a bundle of hollow gas exchange fibers extending between the first end and the second end. A radially inward constriction may be selectively formable in the housing between the first end and the second end around the bundle of hollow gas exchange fibers.

In addition or alternatively to any example disclosed herein, the radially inward constriction is configured to move axially between the first end and the second end.

In addition or alternatively to any example disclosed herein, a blood inlet port is disposed proximate the first end and a blood outlet port is disposed proximate the second end.

In addition or alternatively to any example disclosed herein, forming the radially inward constriction causes mixing of blood flowing from the blood inlet port towards the blood outlet port.

In addition or alternatively to any example disclosed herein, moving the radially inward constriction axially between the first end and the second end creates a pressure wave within blood flowing from the blood inlet port towards the blood outlet port.

The above summary of some embodiments, aspects, and/or examples is not intended to describe each disclosed embodiment or every implementation of the present disclosure. The figures and detailed description which follow more particularly exemplify these embodiments.

### BRIEF DESCRIPTION OF THE DRAWINGS

The disclosure may be more completely understood in consideration of the following detailed description in connection with the accompanying drawings, in which:
FIG. 1 is a left perspective view illustrating selected aspects of an oxygenator;
FIG. 2 is a right perspective view illustrating selected aspects of the oxygenator of FIG. 1;
FIG. 3 is a partial cross-sectional view illustrating selected aspects of the oxygenator of FIGS. 1-2;
FIG. 4 is a transverse cross-sectional view of the oxygenator of FIGS. 1-2 taken along the line 4-4 of FIG. 3;
FIGS. 5-9 illustrate an oxygenator having a housing with one or more radially movable flexible portions;
FIGS. 10-13 illustrate an oxygenator having a housing with a plurality of radially movable annular rings;
FIGS. 14-15 illustrate an oxygenator having a flexible housing disposed within a rigid housing;
FIGS. 16-17 illustrate an oxygenator having a flexible housing including an expandable bladder disposed within a rigid housing;
FIGS. 18-19 illustrate an oxygenator having a flexible housing and an axially movable annular ring disposed within a rigid housing;
FIG. 20 illustrates an oxygenator having a flexible housing disposed within a gas exchanger;
FIGS. 21-22 illustrate an oxygenator having a flexible housing including at least one expandable bladder disposed within a gas exchanger; and
FIG. 23 illustrates an oxygenator having a flexible housing and an axially movable annular ring disposed within a gas exchanger.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the spirit and scope of the disclosure.

### DETAILED DESCRIPTION

The following description should be read with reference to the drawings, which are not necessarily to scale, wherein like reference numerals indicate like elements throughout the several views. The detailed description and drawings are intended to illustrate example embodiments of the disclosure but not limit the disclosure. Those skilled in the art will recognize that the various elements described and/or shown may be arranged in various combinations and configurations without departing from the scope of the disclosure. However, in the interest of clarity and ease of understanding, every feature and/or element may not be shown in each drawing.

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). In many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5).

Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the disclosure are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "retract", variants thereof, and the like, may be generally considered with respect to the positioning, direction, and/or operation of various elements relative to a user/operator/manipulator of the device, wherein "proximal" and "retract" indicate or refer to closer to or toward the user and "distal" and "advance" indicate or refer to farther from or away from the user. In some instances, the terms "proximal" and "distal" may be arbitrarily assigned in an effort to facilitate understanding of the disclosure, and such instances will be readily apparent to the skilled artisan. Still other relative terms, such as "axial", "circumferential", "longitudinal", "lateral", "radial", etc. and/or variants thereof generally refer to direction and/or orientation relative to a central longitudinal axis of the disclosed structure or device.

The term "extent" may be understood to mean the greatest measurement of a stated or identified dimension, unless the extent or dimension in question is preceded by or identified as a "minimum", which may be understood to mean the smallest measurement of the stated or identified dimension. For example, "outer extent" may be understood to mean an outer dimension, "radial extent" may be understood to mean a radial dimension, "longitudinal extent" may be understood to mean a longitudinal dimension, etc. Each instance of an "extent" may be different (e.g., axial, longitudinal, lateral, radial, circumferential, etc.) and will be apparent to the skilled person from the context of the individual usage. Generally, an "extent" may be considered the greatest possible dimension measured according to the intended usage, while a "minimum extent" may be considered the smallest possible dimension measured according to the intended usage. In some instances, an "extent" may generally be measured orthogonally within a plane and/or cross-section, but may be, as will be apparent from the particular context, measured differently - such as, but not limited to, angularly, radially, circumferentially (e.g., along an arc), etc.

The terms "monolithic" and "unitary" shall generally refer to an element or elements made from or consisting of a single structure or base unit/element. A monolithic and/or unitary element shall exclude structure and/or features made by assembling or otherwise joining multiple discrete structures or elements together.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to use the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For example, a reference to some features may be equally referred to all instances and quantities beyond one of said feature(s) unless clearly stated to the contrary. As such, it will be understood that the following discussion may apply equally to any and/or all of the components for which there are more than one within the device, etc. unless explicitly stated to the contrary.

Additionally, it should be noted that in any given figure, some features may not be shown, or may be shown schematically, for clarity and/or simplicity. Additional details regarding some components and/or method steps may be illustrated in other figures in greater detail. The devices and/or methods disclosed herein may provide a number of desirable features and benefits as described in more detail below.

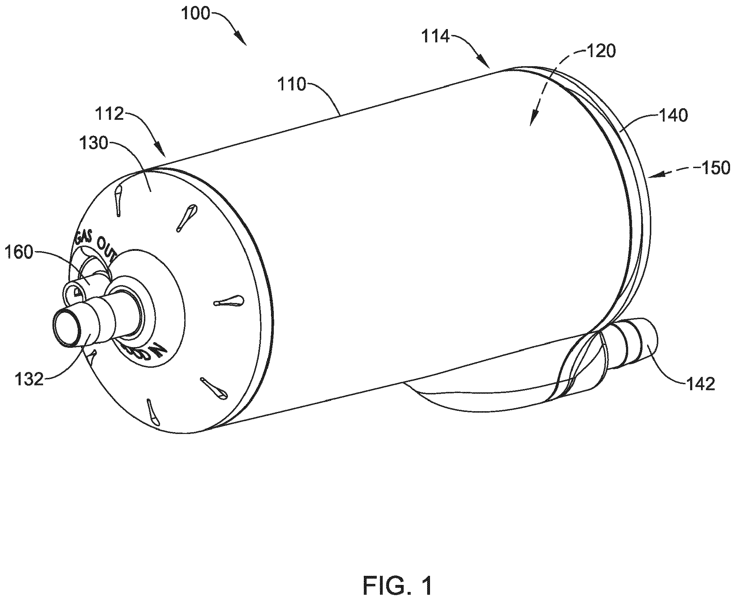

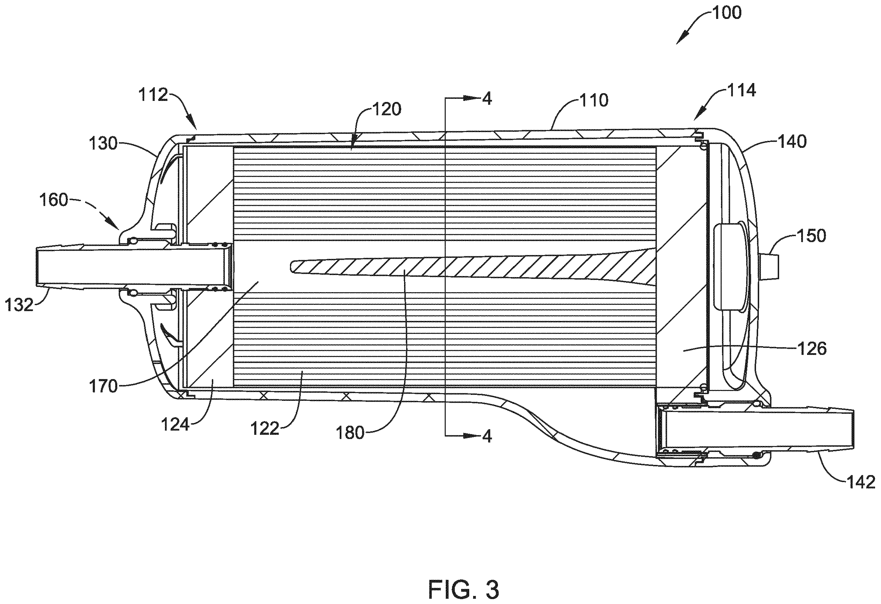

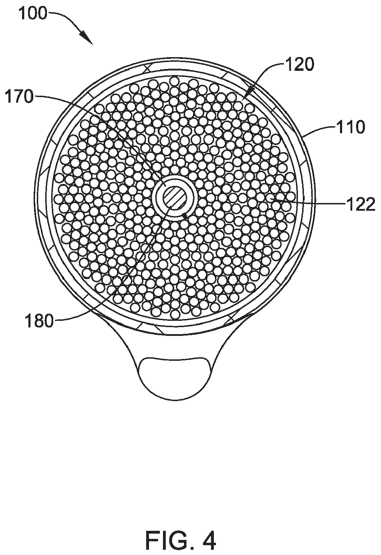

FIGS. 1-4 illustrate selected aspects of an oxygenator 100 for conditioning blood in extracorporeal circulation, such as with an extracorporeal life support (ECLS) system, for example. In some embodiments, the oxygenator 100 may comprise a housing 110 extending from a first end 112 to a second end 114 opposite the first end 112. In at least some embodiments, the housing 110 may be a rigid housing and/or the housing 110 may be constructed from a substantially rigid material. In some embodiments, the housing 110 may be elongated in shape, with a central longitudinal axis extending from the first end 112 to the second end 114. In some embodiments, at least a portion of the housing 110 extending along the central longitudinal axis may be substantially cylindrical in shape.

The oxygenator 100 and/or the housing 110 may include a first cover 130 coupled to the first end 112 of the housing 110. In some embodiments, the first cover 130 may be fixedly attached to the housing 110. In some embodiments, the first cover 130 may be fixedly attached at the first end 112 of the housing 110. In some embodiments, the first cover 130 may be integrally and/or monolithically formed with the housing 110 as a single structure. In some alternative embodiments, the first cover 130 may be removable from the housing 110. Other configurations are also contemplated.

The oxygenator 100 and/or the housing 110 may include a second cover 140 coupled to the second end 114 of the housing 110. In some embodiments, the second cover 140 may be fixedly attached to the housing 110. In some embodiments, the second cover 140 may be fixedly attached at the second end 114 of the housing 110. In some embodiments, the second cover 140 may be integrally and/or monolithically formed with the housing 110 as a single structure. In some alternative embodiments, the second cover 140 may be removable from the housing 110. Other configurations are also contemplated.

The oxygenator 100 may comprise a gas exchanger 120 disposed within the housing 110. In some embodiments, the gas exchanger 120 may be disposed between the first end 112 of the housing 110 and the second end 114 of the housing 110. In at least some embodiments, the gas exchanger 120 may include a bundle of hollow gas exchange fibers 122. In some embodiments, the gas exchanger 120 may include a first potting body 124 disposed proximate the first end 112 of the housing 110 and a second potting body 126 disposed proximate the second end 114 of the housing 110. The bundle of hollow gas exchange fibers 122 may extend from the first potting body 124 to the second potting body 126. In some embodiments, the first potting body 124 and/or the second potting body 126 may be formed from resin or a polymeric material. Proximal and/or upstream ends of the bundle of hollow gas exchange fibers 122 may be fixedly attached to and/or may be embedded within the first potting body 124. Distal and/or downstream ends of the bundle of hollow gas exchange fibers 122 may be fixedly attached to and/or may be embedded within the second potting body 126. The gas exchanger 120 may be configured such that blood flow through the gas exchanger 120 may pass over and/or around the bundle of hollow gas exchange fibers 122 to facilitate gas exchange therebetween.

The first cover 130 and the second cover 140 may be disposed on opposite sides of the gas exchanger 120 and/or the bundle of hollow gas exchange fibers 122. The oxygenator 100, the housing 110, and/or the first cover 130 may include a blood inlet port 132 in fluid communication with the gas exchanger 120 and configured to connect to an extracorporeal life support system. In some embodiments, the blood inlet port 132 may be configured to be in fluid communication with a patient's venous blood flow. In some embodiments, the blood inlet port 132 may be disposed and/or positioned upstream of the gas exchanger 120. In some embodiments, the blood inlet port 132 may be disposed proximate the first end 112 of the housing 110. In some embodiments, the housing 110 and/or the first cover 130 may include a purging line port through which air can be purged from the oxygenator 100 and/or the housing 110.

The oxygenator 100, the housing 110, and/or the second cover 140 may include a blood outlet port 142 in fluid communication with the gas exchanger 120 and configured to connect to the extracorporeal life support system. In some embodiments, the blood outlet port 142 may be configured to be in fluid communication with a patient's arterial blood flow. In some embodiments, the blood outlet port 142 may be disposed and/or positioned downstream of the gas exchanger 120. In some embodiments, the blood outlet port 142 may be disposed proximate the second end 114 of the housing 110. In at least some embodiments, the blood outlet port 142 may be disposed on an opposite side of the gas exchanger 120 and/or the bundle of hollow gas exchange fibers 122 from the blood inlet port 132.

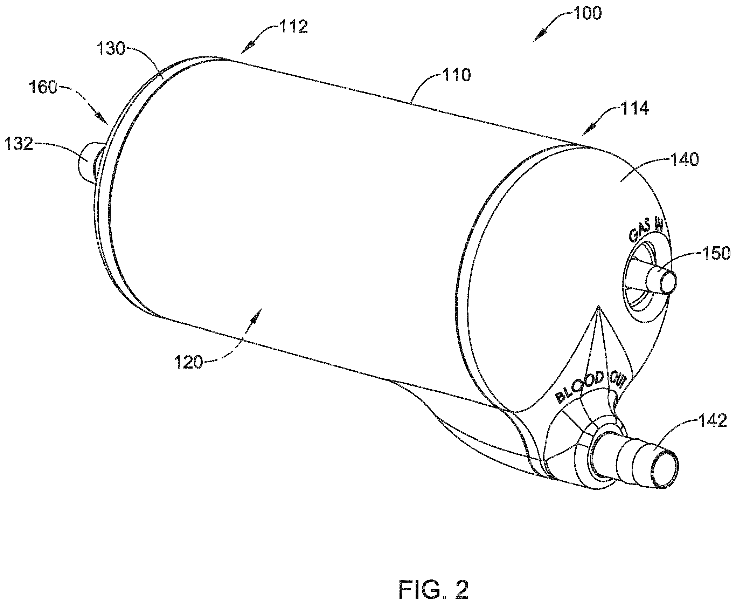

The oxygenator 100 and/or the housing 110 may include a gas inlet port 150 in fluid communication with the gas exchanger 120, the first potting body 124, and/or the bundle of hollow gas exchange fibers 122. In some embodiments, the gas inlet port 150 may be disposed in and/or adjacent to the second cover 140, as seen in FIG. 2. In some embodiments, the gas inlet port 150 may be disposed at a position that is laterally and/or radially offset from the central longitudinal axis of the housing 110. In some embodiments, the gas inlet port 150 may be disposed at a position that is coaxial with the central longitudinal axis of the housing 110. Other configurations are also contemplated. The gas inlet port 150 may be configured to connect to a gas supply source via tubing (not shown) to transport gas (e.g., oxygen, air, etc.) to the oxygenator 100, the gas exchanger 120, and/or the bundle of hollow gas exchange fibers 122.

The oxygenator 100 and/or the housing 110 may include a gas outlet port 160 in fluid communication with the gas exchanger 120, the second potting body 126, and/or the bundle of hollow gas exchange fibers 122. In some embodiments, the gas outlet port 160 may be disposed in and/or adjacent to the first cover 130, as seen in FIG. 1. In some embodiments, the gas outlet port 160 may be disposed at a position that is laterally and/or radially offset from the central longitudinal axis of the housing 110. In some embodiments, the gas outlet port 160 may be disposed at a position that is coaxial with the central longitudinal axis of the housing 110. Other configurations are also contemplated. The gas outlet port 160 may be configured to transport gas (e.g., oxygen, carbon dioxide, air, etc.) away from the oxygenator 100. In some embodiments, the gas outlet port 160 may be in fluid communication with the atmosphere. In some alternative embodiments, the gas outlet port 160 may be configured to connect to a vacuum source, such as via tubing. In some embodiments, the oxygenator 100 and/or the housing 110 may be devoid of a dedicated gas outlet port in fluid communication with the gas exchanger 120, the second potting body 126, and/or the bundle of hollow gas exchange fibers 122. In some embodiments, the oxygenator 100 and/or the housing 110 may include one or more vent openings disposed in and/or adjacent to the first cover or the first end cap 130 and in fluid communication with the gas exchanger 120, the second potting body 126, and/or the bundle of hollow gas exchange fibers 122. In some embodiments, the oxygenator 100 and/or the housing 110 may include the gas outlet port 160 and the one or more vent openings disposed in and/or adjacent to the first cover or the first end cap 130. In some embodiments, the one or more vent openings may be vented to atmosphere.

In some embodiments, the bundle of hollow gas exchange fibers 122 may be disposed within the housing 110. In some embodiments, the bundle of hollow gas exchange fibers 122 may extend between the first end 112 of the housing 110 and the second end 114 of the housing 110. In some embodiments, the bundle of hollow gas exchange fibers 122 may extend longitudinally between the first end 112 of the housing 110 and the second end 114 of the housing 110. In some embodiments, the bundle of hollow gas exchange fibers 122 may extend from the first end 112 of the housing 110 to the second end of the housing 110. As discussed herein, the bundle of hollow gas exchange fibers 122 may extend from the first potting body 124 to the second potting body 126.

The bundle of hollow gas exchange fibers 122 may be arranged in one or more configurations. In some embodiments, the bundle of hollow gas exchange fibers 122 may be arranged such that the bundle of hollow gas exchange fibers 122 is substantially parallel to each other and/or the central longitudinal axis of the housing 110. In some embodiments, the bundle of hollow gas exchange fibers 122 may be arranged such that the bundle of hollow gas exchange fibers 122 is wound around the central longitudinal axis of the housing 110. In some embodiments, the bundle of hollow gas exchange fibers 122 may be arranged such that the bundle of hollow gas exchange fibers 122 is wound helically around the central longitudinal axis of the housing 110. In some embodiments, the bundle of hollow gas exchange fibers 122 may be arranged in a plurality of layers, wherein fibers within each layer are oriented at an oblique angle to fibers within each immediately adjacent layer. In some embodiments, the bundle of hollow gas exchange fibers 122 may be arranged in a plurality of layers, wherein fibers within each layer are oriented substantially perpendicular to fibers within each immediately adjacent layer. Other configurations, including combinations thereof, are also contemplated.

In some embodiments, the oxygenator 100, the housing 110, and/or the gas exchanger 120 may include a blood lumen 170 disposed therein. The blood lumen 170 may be in fluid communication with the blood inlet port 132 and the blood outlet port 142. In at least some embodiments, the blood lumen 170 may be disposed and/or oriented coaxially with the blood inlet port 132. In at least some embodiments, the blood outlet port 142 may be disposed laterally and/or radially offset from the blood lumen 170. The blood lumen 170 may carry and/or direct blood flowing into the oxygenator 100 and/or the housing 110 into and/or through the gas exchanger 120.

In some embodiments, the oxygenator 100 and/or the housing 110 may include a diverter 180 disposed therein. In some embodiments, the diverter 180 may be disposed along and/or parallel to the central longitudinal axis of the housing 110. In some embodiments, the diverter 180 may be disposed in line with and/or coaxial with the blood inlet port 132. In some embodiments, the diverter 180 may be disposed within the blood lumen 170 and/or the gas exchanger 120. The diverter 180 may be configured to disrupt blood flow within the blood lumen 170 and/or to direct blood flowing into the gas exchanger 120 radially outward toward and/or into the bundle of hollow gas exchange fibers 122. In some embodiments, the diverter 180 may extend proximally from the second potting body 126. In some embodiments, the diverter 180 may be tapered radially inward in a proximal direction or an upstream direction and/or may be tapered radially outward in a distal direction or a downstream direction. Other configurations are also contemplated. The diverter 180 may be shaped, sized, and configured to limit damage to blood cells (e.g., lysing) as much as possible. In some embodiments, the diverter 180 may include channels, grooves, ridges, guides, blades, etc. formed in or extending from a generally conical outer surface.

During use, gas may be transferred between the gas exchanger 120 and/or the bundle of hollow gas exchange fibers 122 and blood flowing through the oxygenator 100 and/or the gas exchanger 120 (e.g., oxygen into the blood, carbon dioxide out of the blood, etc.).

In some embodiments, a blood conditioning system and/or the oxygenator 100 may include a pump (not shown) configured to drive fluid flow through the oxygenator 100. In some embodiments, the pump may be fluidly coupled to the oxygenator 100 via tubing. In some embodiments, the pump may extend from the housing 110 and/or the first cover 130 of the oxygenator 100. In some embodiments, the pump may be fixedly attached directly to the oxygenator 100, the housing 110, and/or the first cover 130. In some embodiments, the pump may be integrally formed with the housing 110 and/or the first cover 130 of the oxygenator 100. In some embodiments, the blood conditioning system and/or the oxygenator 100 may be devoid of tubing between and/or connecting the pump and the oxygenator 100. Other configurations are also contemplated.

In some embodiments, the blood conditioning system and/or the oxygenator 100 may include a heat exchanger (not shown) configured to exchange heat with the blood. In some embodiments, the oxygenator 100 and the heat exchanger may be disposed within a single, integrated housing. In some embodiments, the heat exchanger may be a standalone device fluidly coupled to the blood conditioning system and/or the oxygenator 100 via tubing. Other configurations are also contemplated.

In some embodiments, the blood conditioning system and/or the oxygenator 100 may include a gaseous micro-emboli (GME) removal device configured to remove gaseous micro-emboli from the blood. In some embodiments, the oxygenator 100 and the gaseous micro-emboli (GME) removal device may be disposed within a single, integrated housing. In some embodiments, the gaseous micro-emboli (GME) removal device may be a standalone device fluidly coupled to the blood conditioning system and/or the oxygenator 100 via tubing. Other configurations are also contemplated.

While not expressly illustrated, it is also contemplated that the oxygenator 100 may include a cardioplegia port and/or a recirculation port. In some embodiments, the cardioplegia port and/or the recirculation port may be formed in the housing 110. In some embodiments, the cardioplegia port and/or the recirculation port may be formed in and/or may be in fluid communication with the housing 110 and/or an interior thereof. Other configurations and/or features known in the art are also contemplated.

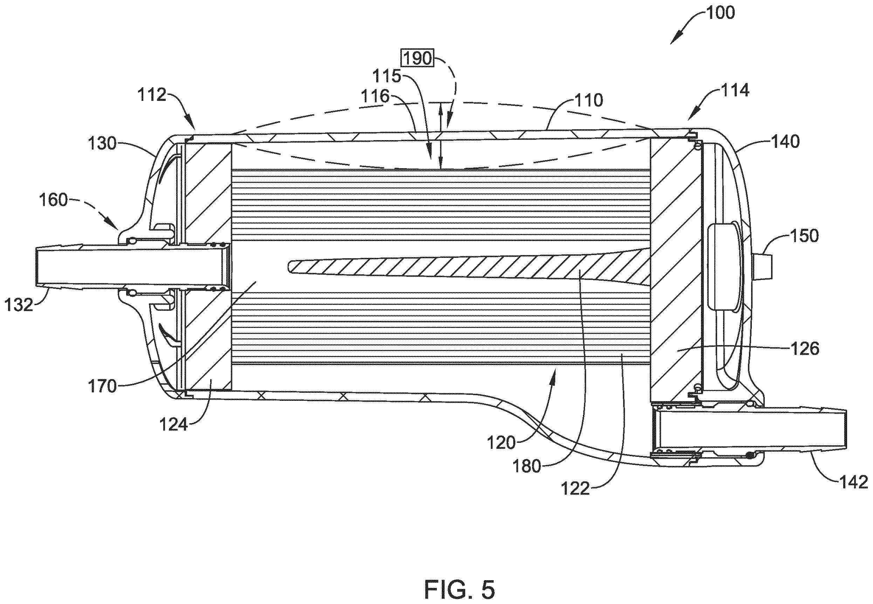

Turning now to FIGS. 5-9, it should be noted that several different configurations for the oxygenator 100 are illustrated. These configurations illustrated may be combined in multiple and/or varying ways so as to create different combinations of the features described herein.

In some embodiments, at least a portion of the housing 110 may be configured to selectively move radially with respect to the gas exchanger 120 and/or the bundle of hollow gas exchange fibers 122. In some embodiments, a wall of the housing 110 extending between the first end 112 of the housing 110 and the second end 114 of the housing 110 may be configured to selectively move radially with respect to the gas exchanger 120 and/or the bundle of hollow gas exchange fibers 122. In some embodiments, the wall of the housing 110 extending between the first end 112 of the housing 110 and the second end 114 of the housing 110 may be configured to shift between radially inward and radially outward positions.

In some embodiments, the housing 110 and/or the wall of the housing 110 may include a flexible portion 116 disposed between the first end 112 of the housing 110 and the second end 114 of the housing 110. The flexible portion 116 may be configured to selectively move radially inward and/or outward relative to and/or with respect to the gas exchanger 120 and/or the bundle of hollow gas exchange fibers 122. The flexible portion 116 may be configured to change and/or disrupt blood flow within the oxygenator 100 and/or the gas exchanger 120 to promote mixing and/or to reduce stagnation, which may reduce or prevent thrombus formation.

In some embodiments, the flexible portion 116 may extend circumferentially around the gas exchanger 120 and/or the bundle of hollow gas exchange fibers 122. In some embodiments, the flexible portion 116 may extend circumferentially around only a portion of the gas exchanger 120 and/or the bundle of hollow gas exchange fibers 122, as seen in FIGS. 5, 6, and 8. In some embodiments, the flexible portion 116 may intermittently and/or interruptedly extend circumferentially around the gas exchanger 120 and/or the bundle of hollow gas exchange fibers 122. In some embodiments, the flexible portion 116 may extend around 75% or less of the circumference of the gas exchanger 120 and/or the bundle of hollow gas exchange fibers 122. In some embodiments, the flexible portion 116 may extend around 50% or less of the circumference of the gas exchanger 120 and/or the bundle of hollow gas exchange fibers 122. In some embodiments, the flexible portion 116 may extend around 25% or less of the circumference of the gas exchanger 120 and/or the bundle of hollow gas exchange fibers 122. Other configurations are also contemplated.

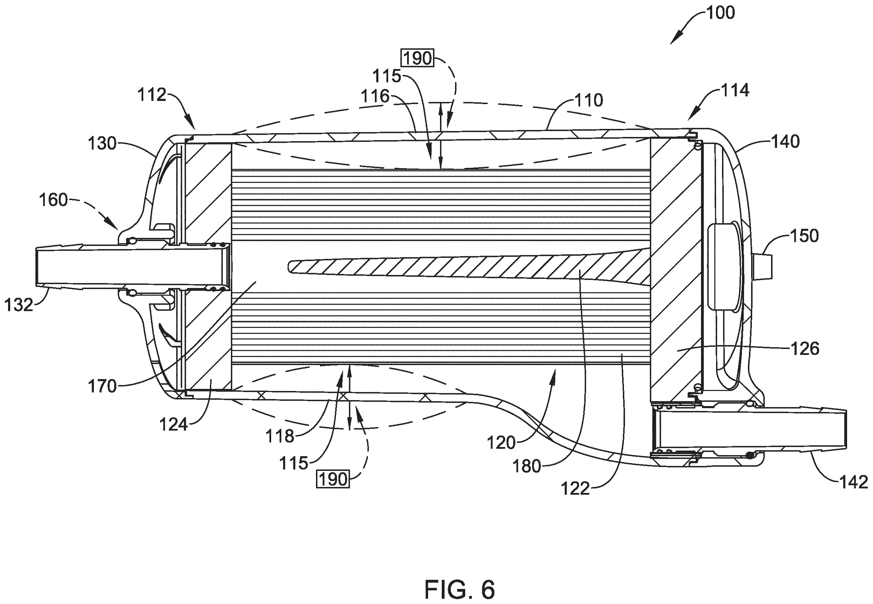

In some embodiments, the flexible portion 116 may extend circumferentially completely around the gas exchanger 120 and/or the bundle of hollow gas exchange fibers 122, as seen in FIGS. 6, 7, and 9. In some embodiments, the flexible portion 116 may extend around an entire circumference of the gas exchanger 120 and/or the bundle of hollow gas exchange fibers 122.

In some embodiments, a radially inward constriction 115 may be selectively formable in the housing 110 and/or the wall of the housing 110 between the first end 112 of the housing 110 and the second end 114 of the housing 110 around the gas exchanger 120 and/or the bundle of hollow gas exchange fibers 122. In some embodiments, the radially inward constriction 115 may cause mixing of blood flowing within the oxygenator 100 and/or the gas exchanger 120 from the blood inlet port 132 towards the blood outlet port 142. In some embodiments, multiple radially inward constrictions may be selectively formable in the housing 110 and/or the wall of the housing 110 between the first end 112 of the housing 110 and the second end 114 of the housing 110 around the gas exchanger 120 and/or the bundle of hollow gas exchange fibers 122.

In some embodiments, the housing 110 and/or the wall of the housing 110 may further include a second flexible portion 118 disposed between the first end 112 and the second end 114, as seen in FIGS. 6-9. The second flexible portion 118 may be configured to selectively move radially inward and/or outward relative to and/or with respect to the gas exchanger 120 and/or the bundle of hollow gas exchange fibers 122. The second flexible portion 118 may be configured to change and/or disrupt blood flow within the oxygenator 100 and/or the gas exchanger 120 to promote mixing and/or to reduce stagnation, which may reduce or prevent thrombus formation. In some embodiments, the flexible portion 116 and the second flexible portion 118 may cooperate with each other to create "waves" (e.g., pressure waves, etc.) within the blood flow within the oxygenator 100 and/or the gas exchanger 120, thereby increasing mixing of blood therein and/or reducing stagnation of blood therein.

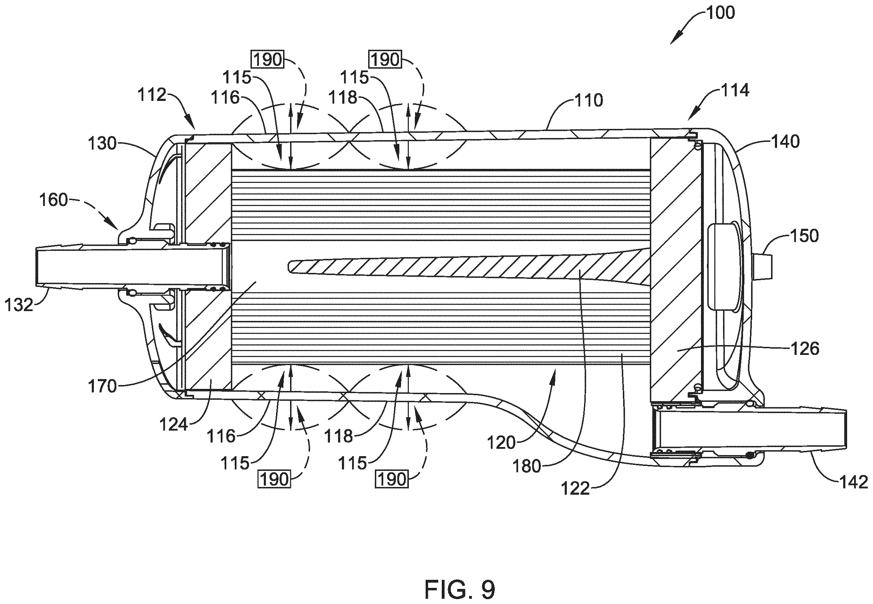

The second flexible portion 118 may be separate and/or distinct from the flexible portion 116. In some embodiments, the second flexible portion 118 may be configured to selectively move radially inward and/or outward relative to and/or with respect to the gas exchanger 120 and/or the bundle of hollow gas exchange fibers 122 independently of the flexible portion 116. In some embodiments, the flexible portion 116 and the second flexible portion 118 may be configured to selectively move radially inward and/or outward relative to and/or with respect to the gas exchanger 120 and/or the bundle of hollow gas exchange fibers 122 sequentially and/or in series with each other. In some embodiments, the second flexible portion 118 may be disposed distal of the flexible portion 116, as seen in FIG. 9. In some embodiments, the second flexible portion 118 may be disposed proximal of the flexible portion 116. In some embodiments, the flexible portion 116 may longitudinally overlap the second flexible portion 118, as seen in FIGS. 6-8.

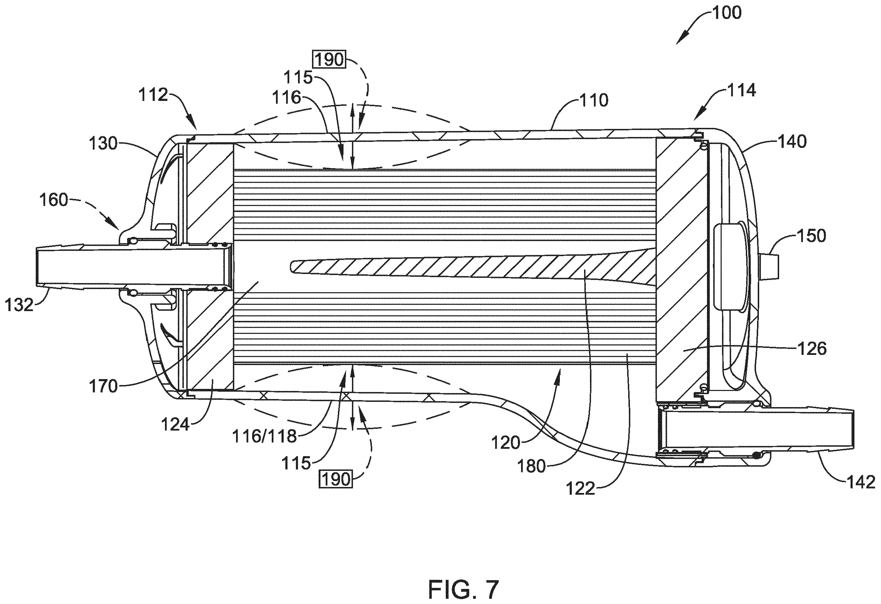

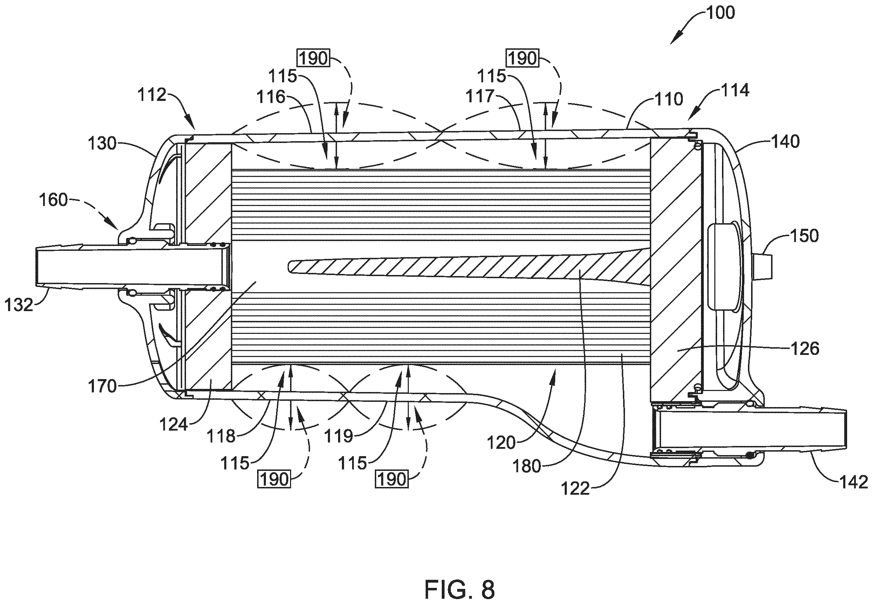

In some embodiments, the flexible portion 116 may have a first longitudinal length and the second flexible portion may have a second longitudinal length. In some embodiments, the first longitudinal length and the second longitudinal length may be different, as seen in FIGS. 6 and 8. In some embodiments, the first longitudinal length may be greater than the second longitudinal length. In some alternative embodiments, the second longitudinal length may be greater than the first longitudinal length. In some embodiments, the first longitudinal length and the second longitudinal length may be the same (e.g., identical), as seen in FIGS. 7 and 9.

In some embodiments, the second flexible portion 118 may extend circumferentially around the gas exchanger 120 and/or the bundle of hollow gas exchange fibers 122. In some embodiments, the second flexible portion 118 may extend circumferentially around only a portion of the gas exchanger 120 and/or the bundle of hollow gas exchange fibers 122, as seen in FIGS. 6-8. In some embodiments, the second flexible portion 118 may intermittently and/or interruptedly extend circumferentially around the gas exchanger 120 and/or the bundle of hollow gas exchange fibers 122. In some embodiments, the second flexible portion 118 may extend around 75% or less of the circumference of the gas exchanger 120 and/or the bundle of hollow gas exchange fibers 122. In some embodiments, the second flexible portion 118 may extend around 50% or less of the circumference of the gas exchanger 120 and/or the bundle of hollow gas exchange fibers 122. In some embodiments, the second flexible portion 118 may extend around 25% or less of the circumference of the gas exchanger 120 and/or the bundle of hollow gas exchange fibers 122. Other configurations are also contemplated.

In some embodiments, the second flexible portion 118 may extend circumferentially completely around the gas exchanger 120 and/or the bundle of hollow gas exchange fibers 122, as seen in FIG. 9. In some embodiments, the flexible portion 116 and/or the second flexible portion 118 may extend around an entire circumference of the gas exchanger 120 and/or the bundle of hollow gas exchange fibers 122.

In some embodiments, the housing 110 and/or the wall of the housing 110 may include additional flexible portions. For example, in some embodiments, the housing 110 and/or the wall of the housing 110 may include a third flexible portion, a fourth flexible portion, a fifth flexible portion, etc. The additional flexible portions (e.g., the third flexible portion, the fourth flexible portion, the fifth flexible portion, etc.) may be configured to selectively move radially with respect to the gas exchanger 120 and/or the bundle of hollow gas exchange fibers 122. In some embodiments, the additional flexible portions (e.g., the third flexible portion, the fourth flexible portion, the fifth flexible portion, etc.) may be configured to selectively move radially with respect to the gas exchanger 120 and/or the bundle of hollow gas exchange fibers 122 in concert with (e.g., simultaneously with) each other. In some embodiments, the additional flexible portions (e.g., the third flexible portion, the fourth flexible portion, the fifth flexible portion, etc.) may be configured to selectively move radially with respect to the gas exchanger 120 and/or the bundle of hollow gas exchange fibers 122 independently of each other. In some embodiments, the additional flexible portions (e.g., the third flexible portion, the fourth flexible portion, the fifth flexible portion, etc.) may be configured to selectively move radially with respect to the gas exchanger 120 and/or the bundle of hollow gas exchange fibers 122 sequentially and/or in series with each other. Other configurations are also contemplated.

In one non-limiting example, the housing 110 and/or the wall of the housing 110 may include secondary flexible portions (e.g., additional flexible portions). As seen in FIG. 8, the flexible portion 116 may include a first secondary flexible portion 117 disposed adjacent thereto and the second flexible portion 118 may include a second secondary flexible portion 119 disposed adjacent thereto. In some embodiments, the first secondary flexible portion 117 may be considered the third flexible portion and/or the second secondary flexible portion 119 may be considered the fourth flexible portion. However, the stated numbering is given merely for identification purposes and is not intended to be limiting. In some embodiments, the flexible portion 116 and the first secondary flexible portion 117 may be configured to selectively move radially with respect to the gas exchanger 120 and/or the bundle of hollow gas exchange fibers 122 sequentially and/or in series with each other. In some embodiments, the second flexible portion 118 and the second secondary flexible portion 119 may be configured to selectively move radially with respect to the gas exchanger 120 and/or the bundle of hollow gas exchange fibers 122 sequentially and/or in series with each other. Other configurations are also contemplated.

In some embodiments, the radially inward constriction 115 may be configured to move axially between the first end 112 of the housing 110 and the second end 114 of the housing 110 around the gas exchanger 120 and/or the bundle of hollow gas exchange fibers 122. In some embodiments, multiple radially inward constrictions may cooperate to move the radially inward constriction 115 axially between the first end 112 of the housing 110 and the second end 114 of the housing 110 around the gas exchanger 120 and/or the bundle of hollow gas exchange fibers 122. In some embodiments, the radially inward constriction 115 may cause mixing of blood flowing within the oxygenator 100 and/or the gas exchanger 120 from the blood inlet port 132 towards the blood outlet port 142. In some embodiments, moving the radially inward constriction 115 axially between the first end 112 of the housing 110 and the second end 114 of the housing 110 around the gas exchanger 120 and/or the bundle of hollow gas exchange fibers 122 may create a wave (e.g., a pressure wave, etc.) within the blood flow within the oxygenator 100 and/or the gas exchanger 120 and/or within blood flowing from the blood inlet port 132 towards the blood outlet port 142, thereby increasing mixing of blood therein and/or reducing stagnation of blood therein.

In some embodiments, the housing 110 may include an actuation apparatus 190 configured to selectively move radially with respect to the gas exchanger 120 and/or the bundle of hollow gas exchange fibers 122. In some embodiments, the housing 110 may include an actuation apparatus 190 configured to selectively move the flexible portion 116 and/or the second flexible portion 118 radially with respect to the gas exchanger 120 and/or the bundle of hollow gas exchange fibers 122. In some embodiments, the actuation apparatus 190 may be configured to operate in a cyclical manner. In some embodiments, the actuation apparatus 190 may be configured to operate in a variable manner. For the purpose of illustration, the actuation apparatus 190 is shown in some figures as a schematic "black box". The skilled artisan will recognize that various changes in form and/or structure may be made depending on the exact mechanism and/or principle of operation of the actuation apparatus 190.

Various configurations and/or types of actuation apparatuses are contemplated in conjunction with the disclosure. In some embodiments, the actuation apparatus 190 may include a shape memory material configured to move and/or shift between a radially inward position and a radially outward position via changes in ambient temperature or another stimulus. In some embodiments, the actuation apparatus 190 may include an electrically sensitive material configured to move and/or shift between a radially inward position and a radially outward position via changes in an applied electrical current and/or electrical resistance. In some embodiments, the actuation apparatus 190 may be magnetically driven and/or may be configured to move and/or shift between a radially inward position and a radially outward position in response to an applied magnetic field. In some embodiments, the actuation apparatus 190 may include a mechanical structure configured to move and/or shift between a radially inward position and a radially outward position. In some embodiments, the mechanical structure may be motor driven. Other configurations are also contemplated. In some embodiments, the actuation apparatus 190 may include an expandable structure configured to move and/or shift between a radially inward position and a radially outward position. In some embodiments, the expandable structure may be configured to move and/or shift between the radially inward position and the radially outward position via changes in fluid pressure and/or volume therein. Other configurations are also contemplated.

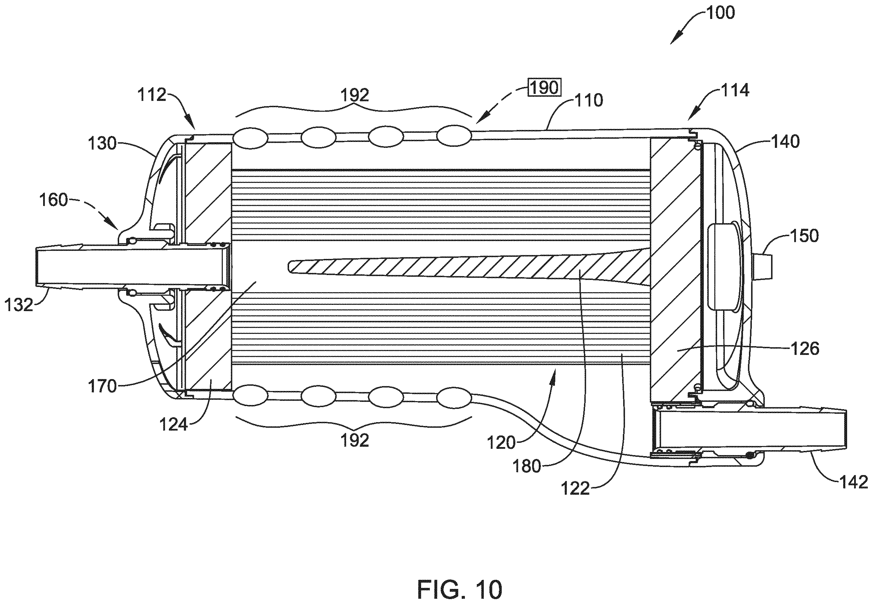

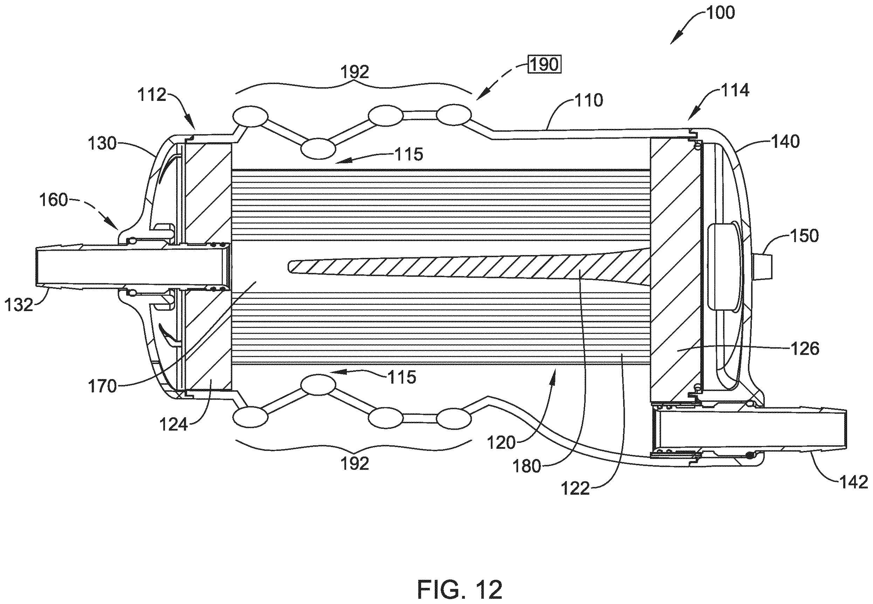

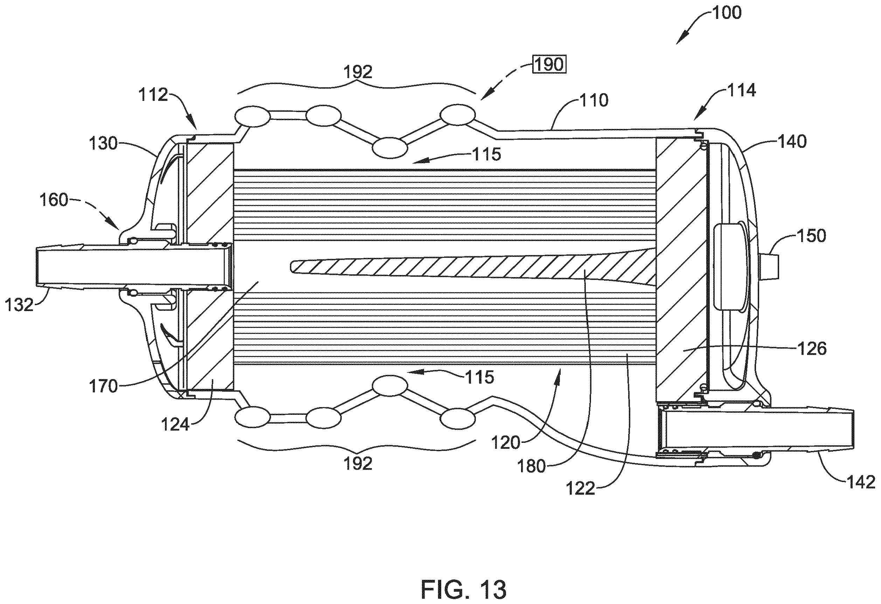

In some embodiments, the actuation apparatus 190 may comprise a plurality of rings 192 disposed between the first end 112 of the housing 110 and the second end 114 of the housing 110, as seen in FIGS. 10-13. In at least some embodiments, each ring of the plurality of rings 192 may be substantially annular.

In some embodiments, each ring of the plurality of rings 192 may be configured to move and/or shift between a radially inward position and a radially outward position. In some embodiments, each ring of the plurality of rings 192 may be configured to move and/or shift radially inward and/or radially outward relative to and/or with respect to the gas exchanger 120 and/or the bundle of hollow gas exchange fibers 122.

In some embodiments, each ring of the plurality of rings 192 may each move and/or shift radially inward and/or radially outward relative to and/or with respect to the gas exchanger 120 and/or the bundle of hollow gas exchange fibers 122 by a common amount or the same amount. In some embodiments, each ring of the plurality of rings 192 may each move and/or shift radially inward and/or radially outward relative to and/or with respect to the gas exchanger 120 and/or the bundle of hollow gas exchange fibers 122 by a different amount.

In some embodiments, the plurality of rings 192 may act together in concert to function and/or act similar to the actuation apparatus 190 above to move the flexible portion 116 and/or the second flexible portion 118 radially inward and/or radially outward relative to and/or with respect to the gas exchanger 120 and/or the bundle of hollow gas exchange fibers 122 in the same manner or in a similar manner to that shown in FIGS. 5-9. To achieve this, rings located toward and/or at proximal and/or distal ends of the plurality of rings 192 may move and/or shift radially inward and/or radially outward relative to and/or with respect to the gas exchanger 120 and/or the bundle of hollow gas exchange fibers 122 less than rings located toward a middle of the plurality of rings 192. In one nonlimiting example using three rings in the plurality of rings 192, a first proximalmost ring may expand radially outward about 25% while a second ring immediately distal of the first proximalmost ring may expand radially outward about 50% while a third distalmost ring immediately distal of the second ring may expand radially outward about 25%. The skilled artisan will recognize that the use of additional rings may vary and/or change expansion and/or contraction amounts within a given ring based on the desired performance.

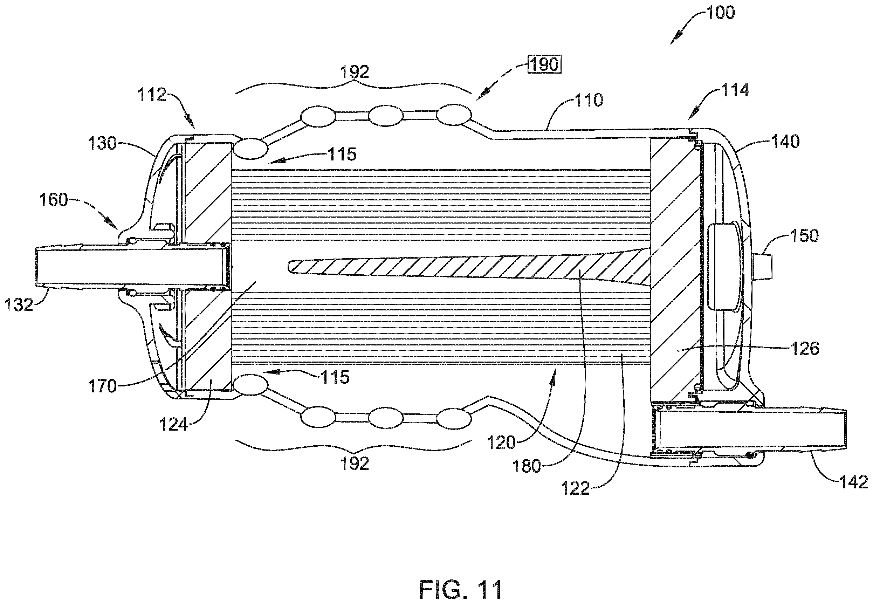

In some embodiments, the plurality of rings 192 may function and/or act to move the flexible portion 116 radially inward and/or radially outward relative to and/or with respect to the gas exchanger 120 and/or the bundle of hollow gas exchange fibers 122 independently of each other. In some embodiments, at least one ring of the plurality of rings 192 may move and/or shift radially inward and/or radially outward relative to and/or with respect to the gas exchanger 120 and/or the bundle of hollow gas exchange fibers 122, while some or all remaining rings of the plurality of rings 192 may not move at all or may move and/or shift radially inward and/or radially outward relative to and/or with respect to the gas exchanger 120 and/or the bundle of hollow gas exchange fibers 122 opposite the at least one ring of the plurality of rings 192. For example, if at least one ring of the plurality of rings 192 moves and/or shifts radially inward relative to and/or with respect to the gas exchanger 120 and/or the bundle of hollow gas exchange fibers 122, some or all remaining rings of the plurality of rings 192 may not move at all or may move and/or shift radially outward relative to and/or with respect to the gas exchanger 120 and/or the bundle of hollow gas exchange fibers 122, as seen in FIGS. 11-13. In another example, if at least one ring of the plurality of rings 192 moves and/or shifts radially outward relative to and/or with respect to the gas exchanger 120 and/or the bundle of hollow gas exchange fibers 122, some or all remaining rings of the plurality of rings 192 may not move at all or may move and/or shift radially inward relative to and/or with respect to the gas exchanger 120 and/or the bundle of hollow gas exchange fibers 122.

In some embodiments, the plurality of rings 192 may function and/or act to move the flexible portion 116 radially inward and/or radially outward relative to and/or with respect to the gas exchanger 120 and/or the bundle of hollow gas exchange fibers 122 sequentially and/or in series to generate waves within the oxygenator 100 and/or the housing 110. For example, FIGS. 10-13 may be considered to illustrate sequential actuation and/or movement of the plurality of rings 192 radially inward and/or radially outward, which may cause a wave or waves to be generated within the oxygenator 100 and/or the housing 110 to promote mixing of blood therein.

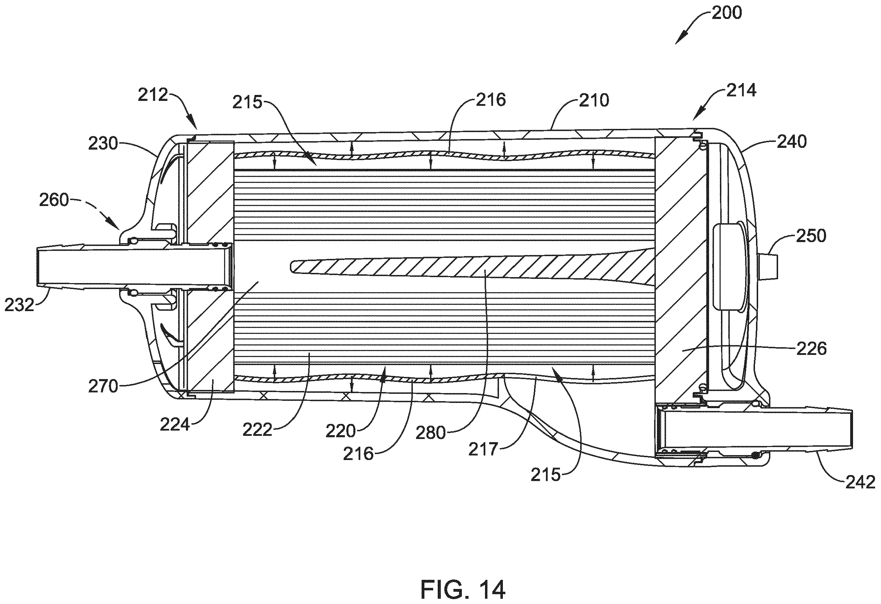

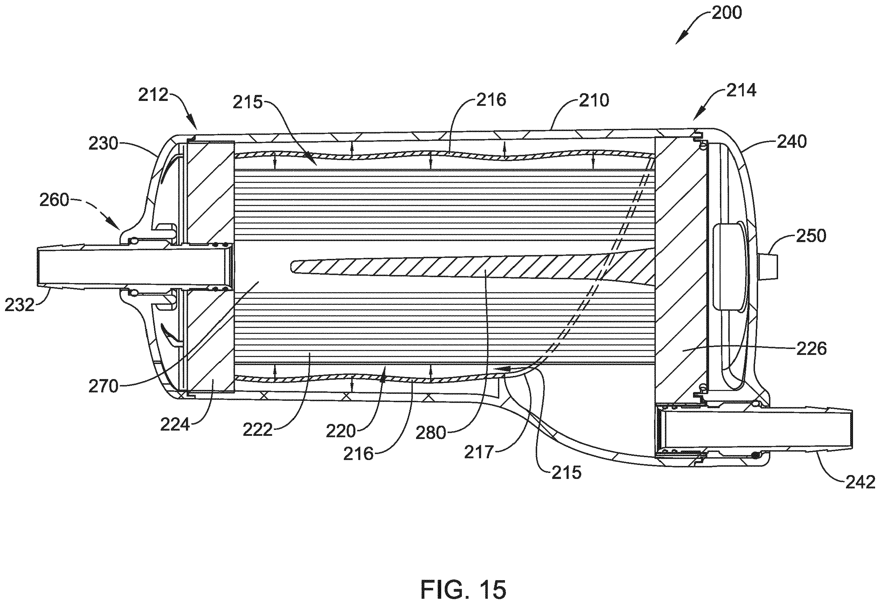

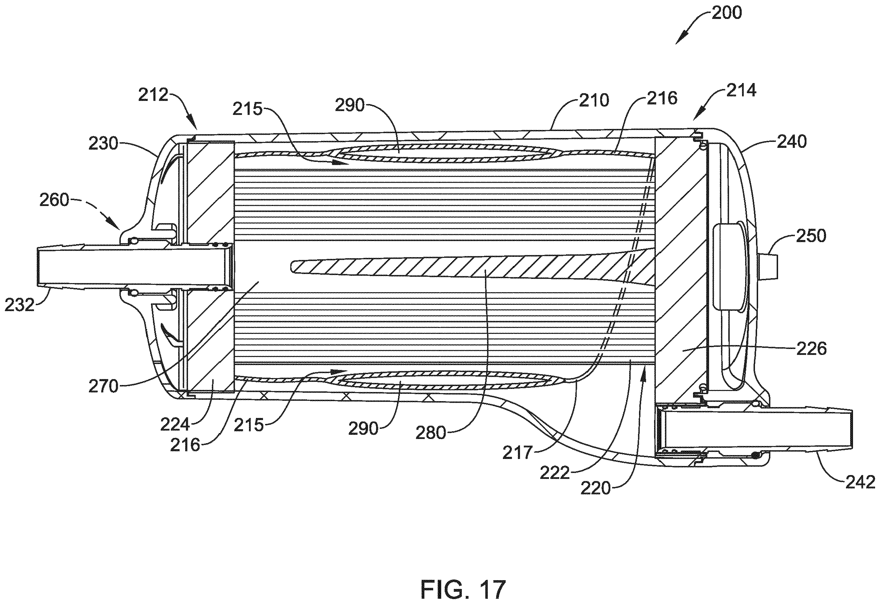

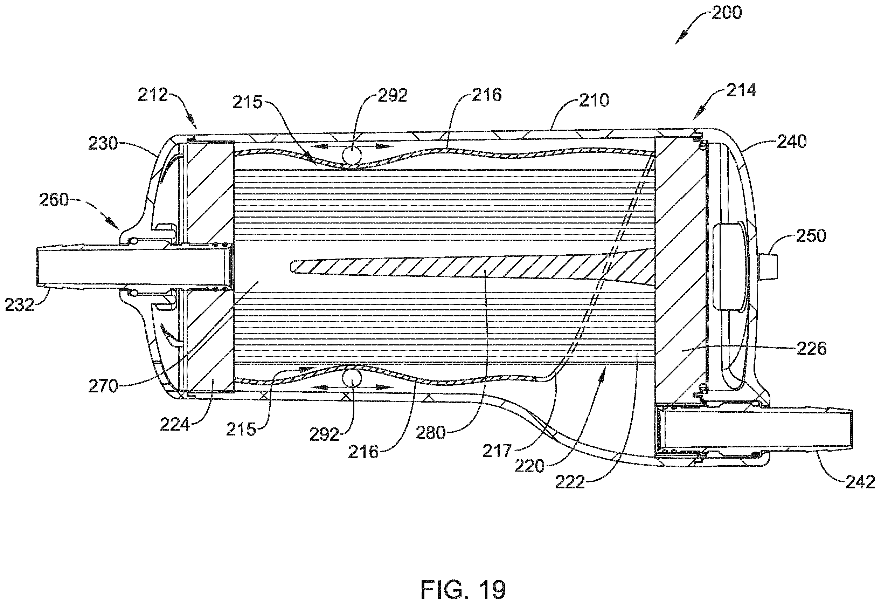

FIGS. 14-19 illustrate selected aspects of an oxygenator 200 for conditioning blood in extracorporeal circulation, such as with an extracorporeal life support (ECLS) system, for example. In some embodiments, an oxygenator 200 may comprise a rigid housing 210 extending from a first end 212 to a second end 214. In some embodiments, the rigid housing 210 may be constructed from a substantially rigid material. In some embodiments, the rigid housing 210 may be elongated in shape, with a central longitudinal axis extending from the first end 212 to the second end 214. In some embodiments, at least a portion of the rigid housing 210 extending along the central longitudinal axis may be substantially cylindrical in shape.

The oxygenator 200 and/or the rigid housing 210 may include a first cover 230 coupled to the first end 212 of the rigid housing 210. In some embodiments, the first cover 230 may be fixedly attached to the rigid housing 210. In some embodiments, the first cover 230 may be fixedly attached at the first end 212 of the rigid housing 210. In some embodiments, the first cover 230 may be integrally and/or monolithically formed with the rigid housing 210 as a single structure. In some alternative embodiments, the first cover 230 may be removable from the rigid housing 210. Other configurations are also contemplated.

The oxygenator 200 and/or the rigid housing 210 may include a second cover 240 coupled to the second end 214 of the rigid housing 210. In some embodiments, the second cover 240 may be fixedly attached to the rigid housing 210. In some embodiments, the second cover 240 may be fixedly attached at the second end 214 of the rigid housing 210. In some embodiments, the second cover 240 may be integrally and/or monolithically formed with the rigid housing 210 as a single structure. In some alternative embodiments, the second cover 240 may be removable from the rigid housing 210. Other configurations are also contemplated.

The oxygenator 200 may comprise a gas exchanger 220 disposed within the rigid housing 210. In some embodiments, the gas exchanger 220 may be disposed between the first end 212 of the rigid housing 210 and the second end 214 of the rigid housing 210. In at least some embodiments, the gas exchanger 220 may include a bundle of hollow gas exchange fibers 222. In some embodiments, the gas exchanger 220 may include a first potting body 224 disposed proximate the first end 212 of the rigid housing 210 and a second potting body 226 disposed proximate the second end 214 of the rigid housing 210. The bundle of hollow gas exchange fibers 222 may extend from the first potting body 224 to the second potting body 226. In some embodiments, the first potting body 224 and/or the second potting body 226 may be formed from resin or a polymeric material. Proximal and/or upstream ends of the bundle of hollow gas exchange fibers 222 may be fixedly attached to and/or may be embedded within the first potting body 224. Distal and/or downstream ends of the bundle of hollow gas exchange fibers 222 may be fixedly attached to and/or may be embedded within the second potting body 226. The gas exchanger 220 may be configured such that blood flow through the gas exchanger 220 may pass over and/or around the bundle of hollow gas exchange fibers 222 to facilitate gas exchange therebetween.

The first cover 230 and the second cover 240 may be disposed on opposite sides of the gas exchanger 220 and/or the bundle of hollow gas exchange fibers 222. The oxygenator 200, the rigid housing 210, and/or the first cover 230 may include a blood inlet port 232 in fluid communication with the gas exchanger 220 and configured to connect to an extracorporeal life support system. In some embodiments, the blood inlet port 232 may be configured to be in fluid communication with a patient's venous blood flow. In some embodiments, the blood inlet port 232 may be disposed and/or positioned upstream of the gas exchanger 220. In some embodiments, the blood inlet port 232 may be disposed proximate the first end 212 of the rigid housing 210. In some embodiments, the rigid housing 210 and/or the first cover 230 may include a purging line port through which air can be purged from the oxygenator 200 and/or the rigid housing 210.

The oxygenator 200, the rigid housing 210, and/or the second cover 240 may include a blood outlet port 242 in fluid communication with the gas exchanger 220 and configured to connect to the extracorporeal life support system. In some embodiments, the blood outlet port 242 may be configured to be in fluid communication with a patient's arterial blood flow. In some embodiments, the blood outlet port 242 may be disposed and/or positioned downstream of the gas exchanger 220. In some embodiments, the blood outlet port 242 may be disposed proximate the second end 214 of the rigid housing 210. In at least some embodiments, the blood outlet port 242 may be disposed on an opposite side of the gas exchanger 220 and/or the bundle of hollow gas exchange fibers 222 from the blood inlet port 232.

The oxygenator 200 and/or the rigid housing 210 may include a gas inlet port 250 in fluid communication with the gas exchanger 220, the first potting body 224, and/or the bundle of hollow gas exchange fibers 222. In some embodiments, the gas inlet port 250 may be disposed in and/or adjacent to the second cover 240. In some embodiments, the gas inlet port 250 may be disposed at a position that is laterally and/or radially offset from the central longitudinal axis of the rigid housing 210. In some embodiments, the gas inlet port 250 may be disposed at a position that is coaxial with the central longitudinal axis of the rigid housing 210. Other configurations are also contemplated. The gas inlet port 250 may be configured to connect to a gas supply source via tubing (not shown) to transport gas (e.g., oxygen, air, etc.) to the oxygenator 200, the gas exchanger 220, and/or the bundle of hollow gas exchange fibers 222.

The oxygenator 200 and/or the rigid housing 210 may include a gas outlet port 260 in fluid communication with the gas exchanger 220, the second potting body 226, and/or the bundle of hollow gas exchange fibers 222. In some embodiments, the gas outlet port 260 may be disposed in and/or adjacent to the first cover 230. It is noted that the gas outlet port 260 is not expressly visible in the figures. However, it is contemplated that the gas outlet port 260 may be similar in form, function, and/or location to the gas outlet port 160 described above. In some embodiments, the gas outlet port 260 may be disposed at a position that is laterally and/or radially offset from the central longitudinal axis of the rigid housing 210. In some embodiments, the gas outlet port 260 may be disposed at a position that is coaxial with the central longitudinal axis of the rigid housing 210. Other configurations are also contemplated. The gas outlet port 260 may be configured to transport gas (e.g., oxygen, carbon dioxide, air, etc.) away from the oxygenator 200. In some embodiments, the gas outlet port 260 may be in fluid communication with the atmosphere. In some alternative embodiments, the gas outlet port 260 may be configured to connect to a vacuum source, such as via tubing. In some embodiments, the oxygenator 200 and/or the housing 210 may be devoid of a dedicated gas outlet port in fluid communication with the gas exchanger 220, the second potting body 226, and/or the bundle of hollow gas exchange fibers 222. In some embodiments, the oxygenator 200 and/or the housing 210 may include one or more vent openings disposed in and/or adjacent to the first cover or the first end cap 230 and in fluid communication with the gas exchanger 220, the second potting body 226, and/or the bundle of hollow gas exchange fibers 222. In some embodiments, the oxygenator 200 and/or the housing 210 may include the gas outlet port 260 and the one or more vent openings disposed in and/or adjacent to the first cover or the first end cap 230. In some embodiments, the one or more vent openings may be vented to atmosphere.

In some embodiments, the bundle of hollow gas exchange fibers 222 may be disposed within the rigid housing 210. In some embodiments, the bundle of hollow gas exchange fibers 222 may extend between the first end 212 of the rigid housing 210 and the second end 214 of the rigid housing 210. In some embodiments, the bundle of hollow gas exchange fibers 222 may extend longitudinally between the first end 212 of the rigid housing 210 and the second end 214 of the rigid housing 210. In some embodiments, the bundle of hollow gas exchange fibers 222 may extend from the first end 212 of the rigid housing 210 to the second end of the rigid housing 210. As discussed herein, the bundle of hollow gas exchange fibers 222 may extend from the first potting body 224 to the second potting body 226.

The bundle of hollow gas exchange fibers 222 may be arranged in one or more configurations. In some embodiments, the bundle of hollow gas exchange fibers 222 may be arranged such that the bundle of hollow gas exchange fibers 222 is substantially parallel to each other and/or the central longitudinal axis of the rigid housing 210. In some embodiments, the bundle of hollow gas exchange fibers 222 may be arranged such that the bundle of hollow gas exchange fibers 222 is wound around the central longitudinal axis of the rigid housing 210. In some embodiments, the bundle of hollow gas exchange fibers 222 may be arranged such that the bundle of hollow gas exchange fibers 222 is wound helically around the central longitudinal axis of the rigid housing 210. In some embodiments, the bundle of hollow gas exchange fibers 222 may be arranged in a plurality of layers, wherein fibers within each layer are oriented at an oblique angle to fibers within each immediately adjacent layer. In some embodiments, the bundle of hollow gas exchange fibers 222 may be arranged in a plurality of layers, wherein fibers within each layer are oriented substantially perpendicular to fibers within each immediately adjacent layer. Other configurations, including combinations thereof, are also contemplated.

In some embodiments, the oxygenator 200, the rigid housing 210, and/or the gas exchanger 220 may include a blood lumen 270 disposed therein. The blood lumen 270 may be in fluid communication with the blood inlet port 232 and the blood outlet port 242. In at least some embodiments, the blood lumen 270 may be disposed and/or oriented coaxially with the blood inlet port 232. In at least some embodiments, the blood outlet port 242 may be disposed laterally and/or radially offset from the blood lumen 270. The blood lumen 270 may carry and/or direct blood flowing into the oxygenator 200 and/or the rigid housing 210 into and/or through the gas exchanger 220.

In some embodiments, the oxygenator 200 and/or the rigid housing 210 may include a diverter 280 disposed therein. In some embodiments, the diverter 280 may be disposed along and/or parallel to the central longitudinal axis of the rigid housing 210. In some embodiments, the diverter 280 may be disposed in line with and/or coaxial with the blood inlet port 232. In some embodiments, the diverter 280 may be disposed within the blood lumen 270 and/or the gas exchanger 220. The diverter 280 may be configured to disrupt blood flow within the blood lumen 270 and/or to direct blood flowing into the gas exchanger 220 radially outward toward and/or into the bundle of hollow gas exchange fibers 222. In some embodiments, the diverter 280 may extend proximally from the second potting body 226. In some embodiments, the diverter 280 may be tapered radially inward in a proximal direction or an upstream direction and/or may be tapered radially outward in a distal direction or a downstream direction. Other configurations are also contemplated. The diverter 280 may be shaped, sized, and configured to limit damage to blood cells (e.g., lysing) as much as possible. In some embodiments, the diverter 280 may include channels, grooves, ridges, guides, blades, etc. formed in or extending from a generally conical outer surface.

During use, gas may be transferred between the gas exchanger 220 and/or the bundle of hollow gas exchange fibers 222 and blood flowing through the oxygenator 200 and/or the gas exchanger 220 (e.g., oxygen into the blood, carbon dioxide out of the blood, etc.).

In some embodiments, a blood conditioning system and/or the oxygenator 200 may include a pump (not shown) configured to drive fluid flow through the oxygenator 200. In some embodiments, the pump may be fluidly coupled to the oxygenator 200 via tubing. In some embodiments, the pump may extend from the rigid housing 210 and/or the first cover 230 of the oxygenator 200. In some embodiments, the pump may be fixedly attached directly to the oxygenator 200, the rigid housing 210, and/or the first cover 230. In some embodiments, the pump may be integrally formed with the rigid housing 210 and/or the first cover 230 of the oxygenator 200. In some embodiments, the blood conditioning system and/or the oxygenator 200 may be devoid of tubing between and/or connecting the pump and the oxygenator 200. Other configurations are also contemplated.

In some embodiments, the blood conditioning system and/or the oxygenator 200 may include a heat exchanger (not shown) configured to exchange heat with the blood. In some embodiments, the oxygenator 200 and the heat exchanger may be disposed within a single, integrated housing. In some embodiments, the heat exchanger may be a standalone device fluidly coupled to the blood conditioning system and/or the oxygenator 200 via tubing. Other configurations are also contemplated.

In some embodiments, the blood conditioning system and/or the oxygenator 200 may include a gaseous micro-emboli (GME) removal device configured to remove gaseous micro-emboli from the blood. In some embodiments, the oxygenator 200 and the gaseous micro-emboli (GME) removal device may be disposed within a single, integrated housing. In some embodiments, the gaseous micro-emboli (GME) removal device may be a standalone device fluidly coupled to the blood conditioning system and/or the oxygenator 200 via tubing. Other configurations are also contemplated.

While not expressly illustrated, it is also contemplated that the oxygenator 200 may include a cardioplegia port and/or a recirculation port. In some embodiments, the cardioplegia port and/or the recirculation port may be formed in the rigid housing 210. In some embodiments, the cardioplegia port and/or the recirculation port may be formed in and/or may be in fluid communication with the flexible housing 216 and/or an interior thereof. Other configurations and/or features known in the art are also contemplated.

The oxygenator 200 and/or the gas exchanger 220 may comprise a flexible housing 216 disposed within the rigid housing 210. In some embodiments, the flexible housing 216 may extend from the first potting body 224 to the second potting body 226. In some embodiments, the flexible housing 216 may extend between the first end 212 of the rigid housing 210 and the second end 214 of the rigid housing 210. In some embodiments, the flexible housing 216 may extend from the first end 212 of the rigid housing 210 to the second end 214 of the rigid housing 210.

The gas exchanger 220 and/or the bundle of hollow gas exchange fibers 222 may be disposed within the flexible housing 216. The gas exchanger 220 and/or the bundle of hollow gas exchange fibers 222 may extend between the first end 212 of the rigid housing 210 and the second end 214 of the rigid housing 210 within the flexible housing 216. In some embodiments, at least a portion of the flexible housing 216 may be configured to selectively move radially with respect to the gas exchanger 220 and/or the bundle of hollow gas exchange fibers 222. In some embodiments, the flexible housing 216 may be configured to shift between radially inward and radially outward positions. The flexible housing 216 may be configured to change and/or disrupt blood flow within the oxygenator 200 and/or the gas exchanger 220 to promote mixing and/or to reduce stagnation, which may reduce or prevent thrombus formation. In some embodiments, movement and/or shifting of the flexible housing 216 radially with respect to the gas exchanger 220 and/or the bundle of hollow gas exchange fibers 222 may create "waves" (e.g., pressure waves, etc.) within the blood flow within the oxygenator 200 and/or the gas exchanger 220, thereby increasing mixing of blood therein and/or reducing stagnation of blood therein.

In some embodiments, the flexible housing 216 may extend circumferentially around the gas exchanger 220 and/or the bundle of hollow gas exchange fibers 222. In some embodiments, the flexible housing 216 may extend circumferentially around only a portion of the gas exchanger 220 and/or the bundle of hollow gas exchange fibers 222. In some embodiments, the flexible housing 216 may intermittently and/or interruptedly extend circumferentially around the gas exchanger 220 and/or the bundle of hollow gas exchange fibers 222. In some embodiments, the flexible housing 216 may extend around 75% or less of the circumference of the gas exchanger 220 and/or the bundle of hollow gas exchange fibers 222. In some embodiments, the flexible housing 216 may extend around 50% or less of the circumference of the gas exchanger 220 and/or the bundle of hollow gas exchange fibers 222. In some embodiments, the flexible housing 216 may extend around 25% or less of the circumference of the gas exchanger 220 and/or the bundle of hollow gas exchange fibers 222. Other configurations are also contemplated.

In at least some embodiments, the flexible housing 216 may extend circumferentially completely around the gas exchanger 220 and/or the bundle of hollow gas exchange fibers 222. In some embodiments, the flexible housing 216 may extend around an entire circumference of the gas exchanger 220 and/or the bundle of hollow gas exchange fibers 222.

In some alternative embodiments, the oxygenator 200 may comprise a plurality of flexible housings and/or the flexible housing 216 may comprise a plurality of distinct flexible portions disposed along a length of the gas exchanger 220 and/or the bundle of hollow gas exchange fibers 222. In some embodiments, each distinct flexible portion of the plurality of distinct flexible portions may extend circumferentially around only a portion of the gas exchanger 220 and/or the bundle of hollow gas exchange fibers 222. In some embodiments, each distinct flexible portion of the plurality of distinct flexible portions may extend circumferentially completely around the gas exchanger 220 and/or the bundle of hollow gas exchange fibers 222. Other configurations are also contemplated.

In some embodiments, the flexible housing 216 may include one or more openings 217 disposed proximate the second end 214 of the rigid housing 210 and/or the blood outlet port 242. The one or more openings 217 may be configured to permit and/or direct blood flow from the gas exchanger 220 toward the blood outlet port 242. In some embodiments, the one or more openings 217 may extend around the circumference of the flexible housing 216. In some embodiments, the one or more openings 217 may be disposed immediately adjacent to the blood outlet port 242. In some embodiments, the one or more openings 217 may include and/or may be one large opening. In some embodiments, the one or more openings 217 may include and/or may be several small openings. In some embodiments, the one or more openings 217 may be a mixture of large opening(s) and small opening(s). Other configurations are also contemplated.

In some embodiments, the oxygenator 200 may include an actuation apparatus configured to selectively move the flexible housing 216 radially with respect to the gas exchanger 220 and/or the bundle of hollow gas exchange fibers 222. In some embodiments, the actuation apparatus may be configured to move and/or shift the flexible housing 216 between a radially inward position and a radially outward position. In some embodiments, the actuation apparatus may be configured to operate in a cyclical manner. In some embodiments, the actuation apparatus may be configured to operate in a variable manner.

In some embodiments, the actuation apparatus may include a fluid, such as a liquid or a gas, disposed between the rigid housing 210 and an outer surface of the flexible housing 216. In some embodiments, changes in pressure and/or volume of the fluid disposed between the rigid housing 210 and the outer surface of the flexible housing 216 may cause at least a portion of the flexible housing 216 to move and/or shift radially with respect to the gas exchanger 220 and/or the bundle of hollow gas exchange fibers 222, thereby causing and/or increasing the mixing of blood within the flexible housing 216 and/or the gas exchanger 220.

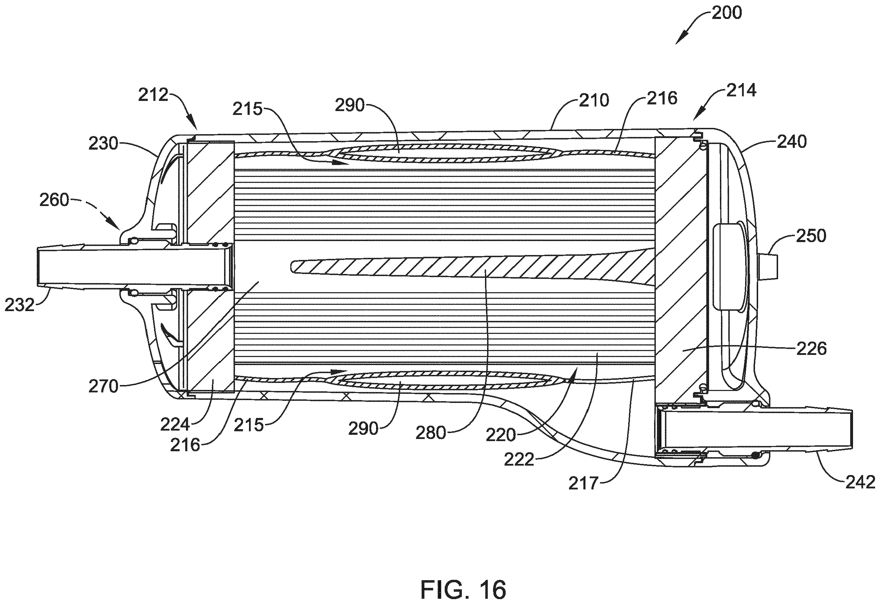

In some embodiments, the actuation apparatus and/or the flexible housing 216 may include an expandable bladder 290 formed within a wall of the flexible housing 216 at a position radially outward of the gas exchanger 220 and/or the bundle of hollow gas exchange fibers 222, as shown in FIGS. 16-17. The expandable bladder 290 may be configured to receive an inflation fluid therein such that changes in pressure or volume of the inflation fluid within the expandable bladder cause at least a portion of the wall of the flexible housing 216 to move and/or shift radially with respect to the gas exchanger 220 and/or the bundle of hollow gas exchange fibers 222. In some embodiments, the inflation fluid may be a liquid (e.g., water, saline, etc.). In some embodiments, the inflation fluid may be a gas (e.g., air, oxygen, etc.). Other configurations are also contemplated. In some embodiments, oxygenator 200 may include an inflation lumen (not shown) fluidly coupled to the expandable bladder 290. In some embodiments, the inflation lumen may be fluidly coupled to a source of inflation fluid (not shown). In some embodiments, the source of inflation fluid may be disposed within the rigid housing 210. In some embodiments, the source of inflation fluid may be disposed external to the rigid housing 210 and fluidly coupled thereto, such as with tubing.

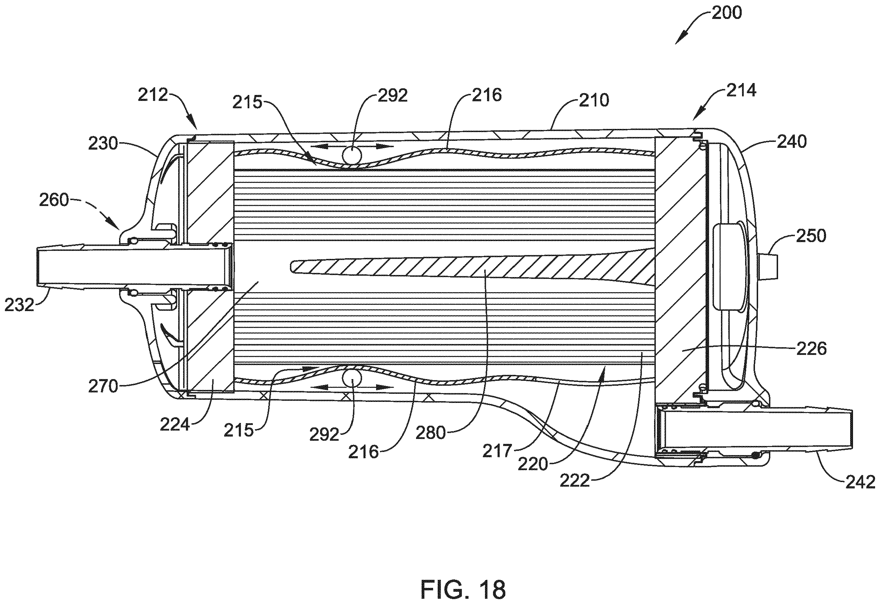

In some embodiments, the oxygenator 200 may comprise an annular ring 292 disposed about and/or around the flexible housing 216, the gas exchanger 220, and/or the bundle of hollow gas exchange fibers 222, as shown in FIGS. 18-19. In some embodiments, the annular ring 292 may be disposed between the rigid housing 210 and the flexible housing 216. The annular ring 292 may have an axial length along the central longitudinal axis of the oxygenator 200 and/or the rigid housing 210 less than a length of the flexible housing 216. In some embodiments, the flexible housing 216 may have a first diameter (e.g., a first radial extent) in an unconstrained configuration. In some embodiments, the flexible housing 216 may be under tension between the first end 212 of the rigid housing 210 and the second end 214 of the rigid housing 210. In some embodiments, the flexible housing 216 may be under tension between the first potting body 224 and the second potting body 226. In some embodiments, tension applied to the flexible housing 216 may generally define the first diameter of the flexible housing 216. In some embodiments, the annular ring 292 may have an inner diameter less than the first diameter of the flexible housing 216 such that the annular ring 292 radially constricts a portion of the flexible housing 216 disposed therein to a second diameter less than the first diameter, as shown in FIGS. 18-19.

In at least some embodiments, the annular ring 292 may be movable axially along the flexible housing 216 and/or within the rigid housing 210. In some embodiments, the annular ring 292 may be movable axially along the flexible housing 216 between the first end 212 of the rigid housing 210 and the second end 214 of the rigid housing 210. As the annular ring 292 moves axially along the flexible housing 216 and/or between the first end 212 of the rigid housing 210 and the second end 214 of the rigid housing 210, the annular ring 292 radially constricts a portion or a subsection of the flexible housing 216. In some embodiments, constricting a portion or a subsection of the flexible housing 216 may create "waves" (e.g., pressure waves, etc.) within the blood flow within the oxygenator 200 and/or the gas exchanger 220, thereby increasing mixing of blood therein and/or reducing stagnation of blood therein.

In some embodiments, a radially inward constriction 215 may be selectively formable in the flexible housing 216 between the first end 212 of the rigid housing 210 and the second end 214 of the rigid housing 210 around the gas exchanger 220 and/or the bundle of hollow gas exchange fibers 222, as seen in FIGS. 14-19. In some embodiments, the radially inward constriction 215 may cause mixing of blood flowing within the oxygenator 200 and/or the gas exchanger 220 from the blood inlet port 232 towards the blood outlet port 242. In some embodiments, multiple radially inward constrictions may be selectively formable in the flexible housing 216 between the first end 212 of the rigid housing 210 and the second end 214 of the rigid housing 210 around the gas exchanger 220 and/or the bundle of hollow gas exchange fibers 222.

In some embodiments, the radially inward constriction 215 may be configured to move axially between the first end 212 of the rigid housing 210 and the second end 214 of the rigid housing 210 around the gas exchanger 220 and/or the bundle of hollow gas exchange fibers 222. In some embodiments, multiple radially inward constrictions may cooperate to move the radially inward constriction 215 axially between the first end 212 of the rigid housing 210 and the second end 214 of the rigid housing 210 around the gas exchanger 220 and/or the bundle of hollow gas exchange fibers 222. In some embodiments, the radially inward constriction 215 may cause mixing of blood flowing within the oxygenator 200 and/or the gas exchanger 220 from the blood inlet port 232 towards the blood outlet port 242. In some embodiments, moving the radially inward constriction 215 axially between the first end 212 of the rigid housing 210 and the second end 214 of the rigid housing 210 around the gas exchanger 220 and/or the bundle of hollow gas exchange fibers 222 may create a wave (e.g., a pressure wave, etc.) within the blood flow within the oxygenator 200 and/or the gas exchanger 220 and/or within blood flowing from the blood inlet port 232 towards the blood outlet port 242, thereby increasing mixing of blood therein and/or reducing stagnation of blood therein.

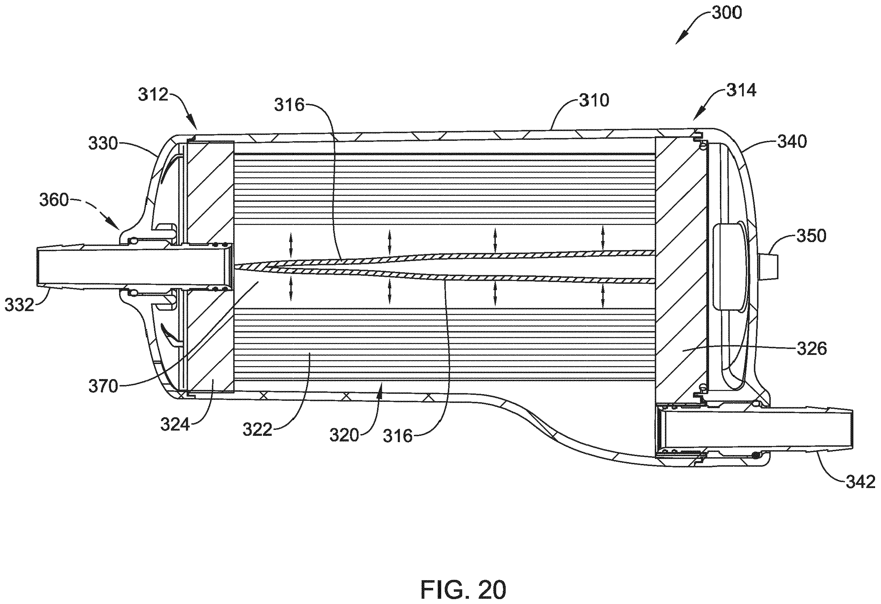

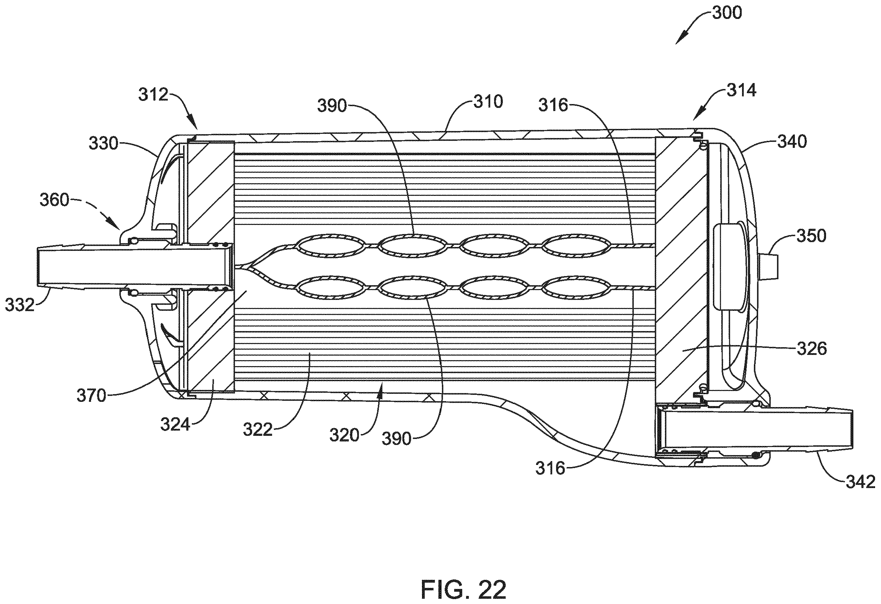

FIGS. 20-23 illustrate selected aspects of an oxygenator 300 for conditioning blood in extracorporeal circulation, such as with an extracorporeal life support (ECLS) system, for example. In some embodiments, the oxygenator 300 may comprise a rigid housing 310 extending from a first end 312 to a second end 314. In some embodiments, the rigid housing 310 may be constructed from a substantially rigid material. In some embodiments, the rigid housing 310 may be elongated in shape, with a central longitudinal axis extending from the first end 312 to the second end 314. In some embodiments, at least a portion of the rigid housing 310 extending along the central longitudinal axis may be substantially cylindrical in shape.

The oxygenator 300 and/or the rigid housing 310 may include a first cover 330 coupled to the first end 312 of the rigid housing 310. In some embodiments, the first cover 330 may be fixedly attached to the rigid housing 310. In some embodiments, the first cover 330 may be fixedly attached at the first end 312 of the rigid housing 310. In some embodiments, the first cover 330 may be integrally and/or monolithically formed with the rigid housing 310 as a single structure. In some alternative embodiments, the first cover 330 may be removable from the rigid housing 310. Other configurations are also contemplated.

The oxygenator 300 and/or the rigid housing 310 may include a second cover 340 coupled to the second end 314 of the rigid housing 310. In some embodiments, the second cover 340 may be fixedly attached to the rigid housing 310. In some embodiments, the second cover 340 may be fixedly attached at the second end 314 of the rigid housing 310. In some embodiments, the second cover 340 may be integrally and/or monolithically formed with the rigid housing 310 as a single structure. In some alternative embodiments, the second cover 340 may be removable from the rigid housing 310. Other configurations are also contemplated.

The oxygenator 300 may comprise a gas exchanger 320 disposed within the rigid housing 310. In some embodiments, the gas exchanger 320 may be disposed between the first end 312 of the rigid housing 310 and the second end 314 of the rigid housing 310. In at least some embodiments, the gas exchanger 320 may include a bundle of hollow gas exchange fibers 322. In some embodiments, the gas exchanger 320 may include a first potting body 324 disposed proximate the first end 312 of the rigid housing 310 and a second potting body 326 disposed proximate the second end 314 of the rigid housing 310. The bundle of hollow gas exchange fibers 322 may extend from the first potting body 324 to the second potting body 326. In some embodiments, the first potting body 324 and/or the second potting body 326 may be formed from resin or a polymeric material. Proximal and/or upstream ends of the bundle of hollow gas exchange fibers 322 may be fixedly attached to and/or may be embedded within the first potting body 324. Distal and/or downstream ends of the bundle of hollow gas exchange fibers 322 may be fixedly attached to and/or may be embedded within the second potting body 326. The gas exchanger 320 may be configured such that blood flow through the gas exchanger 320 may pass over and/or around the bundle of hollow gas exchange fibers 322 to facilitate gas exchange therebetween.

The first cover 330 and the second cover 340 may be disposed on opposite sides of the gas exchanger 320 and/or the bundle of hollow gas exchange fibers 322. The oxygenator 300, the rigid housing 310, and/or the first cover 330 may include a blood inlet port 332 in fluid communication with the gas exchanger 320 and configured to connect to an extracorporeal life support system. In some embodiments, the blood inlet port 332 may be configured to be in fluid communication with a patient's venous blood flow. In some embodiments, the blood inlet port 332 may be disposed and/or positioned upstream of the gas exchanger 320. In some embodiments, the blood inlet port 332 may be disposed proximate the first end 312 of the rigid housing 310. In some embodiments, the rigid housing 310 and/or the first cover 330 may include a purging line port through which air can be purged from the oxygenator 300 and/or the rigid housing 310.

The oxygenator 300, the rigid housing 310, and/or the second cover 340 may include a blood outlet port 342 in fluid communication with the gas exchanger 320 and configured to connect to the extracorporeal life support system. In some embodiments, the blood outlet port 342 may be configured to be in fluid communication with a patient's arterial blood flow. In some embodiments, the blood outlet port 342 may be disposed and/or positioned downstream of the gas exchanger 320. In some embodiments, the blood outlet port 342 may be disposed proximate the second end 314 of the rigid housing 310. In at least some embodiments, the blood outlet port 342 may be disposed on an opposite side of the gas exchanger 320 and/or the bundle of hollow gas exchange fibers 322 from the blood inlet port 332.

The oxygenator 300 and/or the rigid housing 310 may include a gas inlet port 350 in fluid communication with the gas exchanger 320, the first potting body 324, and/or the bundle of hollow gas exchange fibers 322. In some embodiments, the gas inlet port 350 may be disposed in and/or adjacent to the second cover 340. In some embodiments, the gas inlet port 350 may be disposed at a position that is laterally and/or radially offset from the central longitudinal axis of the rigid housing 310. In some embodiments, the gas inlet port 350 may be disposed at a position that is coaxial with the central longitudinal axis of the rigid housing 310. Other configurations are also contemplated. The gas inlet port 350 may be configured to connect to a gas supply source via tubing (not shown) to transport gas (e.g., oxygen, air, etc.) to the oxygenator 300, the gas exchanger 320, and/or the bundle of hollow gas exchange fibers 322.

The oxygenator 300 and/or the rigid housing 310 may include a gas outlet port 360 in fluid communication with the gas exchanger 320, the second potting body 326, and/or the bundle of hollow gas exchange fibers 322. In some embodiments, the gas outlet port 360 may be disposed in and/or adjacent to the first cover 330. It is noted that the gas outlet port 360 is not expressly visible in the figures. However, it is contemplated that the gas outlet port 360 may be similar in form, function, and/or location to the gas outlet port 160/260 described above. In some embodiments, the gas outlet port 360 may be disposed at a position that is laterally and/or radially offset from the central longitudinal axis of the rigid housing 310. In some embodiments, the gas outlet port 360 may be disposed at a position that is coaxial with the central longitudinal axis of the rigid housing 310. Other configurations are also contemplated. The gas outlet port 360 may be configured to transport gas (e.g., oxygen, carbon dioxide, air, etc.) away from the oxygenator 300. In some embodiments, the gas outlet port 360 may be in fluid communication with the atmosphere. In some alternative embodiments, the gas outlet port 360 may be configured to connect to a vacuum source, such as via tubing. In some embodiments, the oxygenator 300 and/or the housing 310 may be devoid of a dedicated gas outlet port in fluid communication with the gas exchanger 320, the second potting body 326, and/or the bundle of hollow gas exchange fibers 322. In some embodiments, the oxygenator 300 and/or the housing 310 may include one or more vent openings disposed in and/or adjacent to the first cover or the first end cap 330 and in fluid communication with the gas exchanger 320, the second potting body 326, and/or the bundle of hollow gas exchange fibers 322. In some embodiments, the oxygenator 300 and/or the housing 310 may include the gas outlet port 360 and the one or more vent openings disposed in and/or adjacent to the first cover or the first end cap 330. In some embodiments, the one or more vent openings may be vented to atmosphere.

In some embodiments, the bundle of hollow gas exchange fibers 322 may be disposed within the rigid housing 310. In some embodiments, the bundle of hollow gas exchange fibers 322 may extend between the first end 312 of the rigid housing 310 and the second end 314 of the rigid housing 310. In some embodiments, the bundle of hollow gas exchange fibers 322 may extend longitudinally between the first end 312 of the rigid housing 310 and the second end 314 of the rigid housing 310. In some embodiments, the bundle of hollow gas exchange fibers 322 may extend from the first end 312 of the rigid housing 310 to the second end of the rigid housing 310. As discussed herein, the bundle of hollow gas exchange fibers 322 may extend from the first potting body 324 to the second potting body 326.

The bundle of hollow gas exchange fibers 322 may be arranged in one or more configurations. In some embodiments, the bundle of hollow gas exchange fibers 322 may be arranged such that the bundle of hollow gas exchange fibers 322 is substantially parallel to each other and/or the central longitudinal axis of the rigid housing 310. In some embodiments, the bundle of hollow gas exchange fibers 322 may be arranged such that the bundle of hollow gas exchange fibers 322 is wound around the central longitudinal axis of the rigid housing 310. In some embodiments, the bundle of hollow gas exchange fibers 322 may be arranged such that the bundle of hollow gas exchange fibers 322 is wound helically around the central longitudinal axis of the rigid housing 310. In some embodiments, the bundle of hollow gas exchange fibers 322 may be arranged in a plurality of layers, wherein fibers within each layer are oriented at an oblique angle to fibers within each immediately adjacent layer. In some embodiments, the bundle of hollow gas exchange fibers 322 may be arranged in a plurality of layers, wherein fibers within each layer are oriented substantially perpendicular to fibers within each immediately adjacent layer. Other configurations, including combinations thereof, are also contemplated.

In some embodiments, the oxygenator 300, the rigid housing 310, and/or the gas exchanger 320 may include a blood lumen 370 disposed therein. The blood lumen 370 may be in fluid communication with the blood inlet port 332 and the blood outlet port 342. In at least some embodiments, the blood lumen 370 may be disposed and/or oriented coaxially with the blood inlet port 332. In at least some embodiments, the blood outlet port 342 may be disposed laterally and/or radially offset from the blood lumen 370. The blood lumen 370 may carry and/or direct blood flowing into the oxygenator 300 and/or the rigid housing 310 into and/or through the gas exchanger 320.

During use, gas may be transferred between the gas exchanger 320 and/or the bundle of hollow gas exchange fibers 322 and blood flowing through the oxygenator 300 and/or the gas exchanger 320 (e.g., oxygen into the blood, carbon dioxide out of the blood, etc.).

In some embodiments, a blood conditioning system and/or the oxygenator 300 may include a pump (not shown) configured to drive fluid flow through the oxygenator 300. In some embodiments, the pump may be fluidly coupled to the oxygenator 300 via tubing. In some embodiments, the pump may extend from the rigid housing 310 and/or the first cover 330 of the oxygenator 300. In some embodiments, the pump may be fixedly attached directly to the oxygenator 300, the rigid housing 310, and/or the first cover 330. In some embodiments, the pump may be integrally formed with the rigid housing 310 and/or the first cover 330 of the oxygenator 300. In some embodiments, the blood conditioning system and/or the oxygenator 300 may be devoid of tubing between and/or connecting the pump and the oxygenator 300. Other configurations are also contemplated.

In some embodiments, the blood conditioning system and/or the oxygenator 300 may include a heat exchanger (not shown) configured to exchange heat with the blood. In some embodiments, the oxygenator 300 and the heat exchanger may be disposed within a single, integrated housing. In some embodiments, the heat exchanger may be a standalone device fluidly coupled to the blood conditioning system and/or the oxygenator 300 via tubing. Other configurations are also contemplated.

In some embodiments, the blood conditioning system and/or the oxygenator 300 may include a gaseous micro-emboli (GME) removal device configured to remove gaseous micro-emboli from the blood. In some embodiments, the oxygenator 300 and the gaseous micro-emboli (GME) removal device may be disposed within a single, integrated housing. In some embodiments, the gaseous micro-emboli (GME) removal device may be a standalone device fluidly coupled to the blood conditioning system and/or the oxygenator 300 via tubing. Other configurations are also contemplated.

While not expressly illustrated, it is also contemplated that the oxygenator 300 may include a cardioplegia port and/or a recirculation port. In some embodiments, the cardioplegia port and/or the recirculation port may be formed in and/or may be in fluid communication with the rigid housing 310 and/or an interior thereof. Other configurations and/or features known in the art are also contemplated.

The oxygenator 300 and/or the gas exchanger 320 may comprise a flexible housing 316 disposed within the gas exchanger 320, the bundle of hollow gas exchange fibers 322, and/or the blood lumen 370. In some embodiments, the flexible housing 316 may extend between the first potting body 324 and the second potting body 326. In some embodiments, the flexible housing 316 may extend from the first potting body 324 to the second potting body 326.

In some embodiments, the flexible housing 316 may be configured to disrupt blood flow within the blood lumen 370 and/or to direct blood flowing into the gas exchanger 320 radially outward toward and/or into the bundle of hollow gas exchange fibers 322. In some embodiments, the flexible housing 316 may extend proximally from the second potting body 226. In some embodiments, the flexible housing 316 may be tapered radially inward in a proximal direction or an upstream direction and/or may be tapered radially outward in a distal direction or a downstream direction. Other configurations are also contemplated. The flexible housing 316 may be shaped, sized, and configured to limit damage to blood cells (e.g., lysing) as much as possible. In some embodiments, the flexible housing 316 may include channels, grooves, ridges, guides, blades, etc. formed in or extending from a generally conical outer surface.

The gas exchanger 320 and/or the bundle of hollow gas exchange fibers 322 may be disposed radially outward of the flexible housing 316. In some embodiments, at least a portion of the flexible housing 316 may be configured to selectively move radially with respect to the gas exchanger 320 and/or the bundle of hollow gas exchange fibers 322. In some embodiments, the flexible housing 316 may be configured to shift between radially inward and radially outward positions. The flexible housing 316 may be configured to change and/or disrupt blood flow within the oxygenator 300 and/or the gas exchanger 320 to promote mixing and/or to reduce stagnation, which may reduce or prevent thrombus formation. In some embodiments, movement and/or shifting of the flexible housing 316 radially with respect to the gas exchanger 320 and/or the bundle of hollow gas exchange fibers 322 may create "waves" (e.g., pressure waves, etc.) within the blood flow within the oxygenator 300 and/or the gas exchanger 320, thereby increasing mixing of blood therein and/or reducing stagnation of blood therein.

In some embodiments, the gas exchanger 320 and/or the bundle of hollow gas exchange fibers 322 may extend circumferentially around the flexible housing 316. In some embodiments, the gas exchanger 320 and/or the bundle of hollow gas exchange fibers 322 may extend circumferentially around only a portion of the flexible housing 316. In some embodiments, the gas exchanger 320 and/or the bundle of hollow gas exchange fibers 322 may intermittently and/or interruptedly extend circumferentially around the flexible housing 316. In some embodiments, the gas exchanger 320 and/or the bundle of hollow gas exchange fibers 322 may extend around 75% or less of the circumference of the flexible housing 316. In some embodiments, the gas exchanger 320 and/or the bundle of hollow gas exchange fibers 322 may extend around 50% or less of the circumference of the flexible housing 316. In some embodiments, the gas exchanger 320 and/or the bundle of hollow gas exchange fibers 322 may extend around 25% or less of the circumference of the flexible housing 316. Other configurations are also contemplated.

In at least some embodiments, the gas exchanger 320 and/or the bundle of hollow gas exchange fibers 322 may extend circumferentially completely around the flexible housing 316. In some embodiments, the gas exchanger 320 and/or the bundle of hollow gas exchange fibers 322 may extend around an entire circumference of the flexible housing 316.

In some alternative embodiments, the oxygenator 300 may comprise a plurality of flexible housings and/or the flexible housing 316 may comprise a plurality of distinct flexible portions disposed along a length of the gas exchanger 320 and/or the bundle of hollow gas exchange fibers 322. Other configurations are also contemplated.

In some embodiments, the oxygenator 300 may include an actuation apparatus configured to selectively move the flexible housing 316 radially with respect to the gas exchanger 320 and/or the bundle of hollow gas exchange fibers 322. In some embodiments, the actuation apparatus may be configured to move and/or shift the flexible housing 316 between a radially inward position and a radially outward position. In some embodiments, the actuation apparatus may be configured to operate in a cyclical manner. In some embodiments, the actuation apparatus may be configured to operate in a variable manner.

In some embodiments, the actuation apparatus may include a fluid, such as a liquid or a gas, disposed within the flexible housing 316. In some embodiments, changes in pressure and/or volume of the fluid disposed within the flexible housing 316 may cause at least a portion of the flexible housing 316 to move and/or shift radially with respect to the gas exchanger 320 and/or the bundle of hollow gas exchange fibers 322, thereby causing and/or increasing the mixing of blood within the oxygenator 300 and/or the gas exchanger 320.

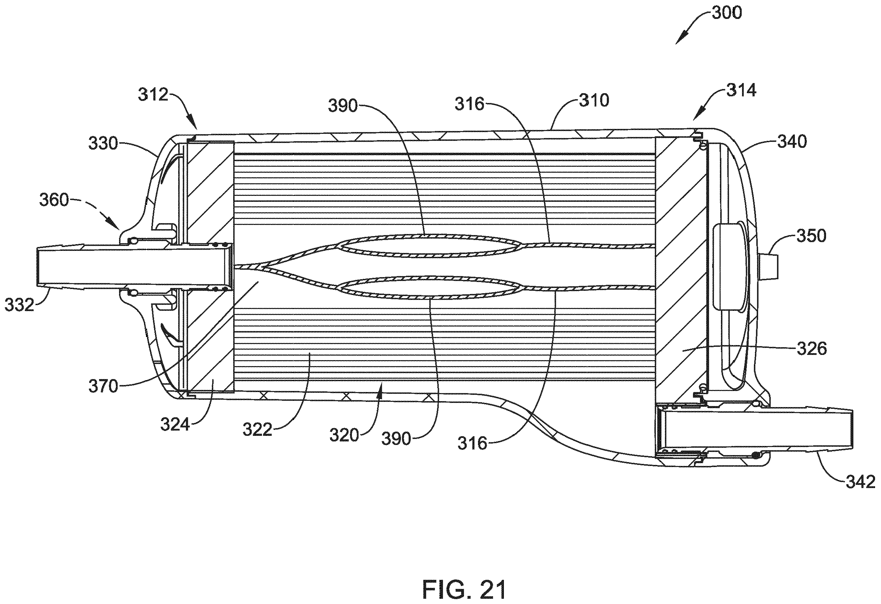

In some embodiments, the actuation apparatus and/or the flexible housing 316 may include at least one expandable bladder 390 formed within a wall of the flexible housing 316 at a position radially inward of the gas exchanger 320 and/or the bundle of hollow gas exchange fibers 322, as shown in FIGS. 21-22. The at least one expandable bladder 390 may be configured to receive an inflation fluid therein such that changes in pressure or volume of the inflation fluid within the at least one expandable bladder 390 cause at least a portion of the wall of the flexible housing 316 to move and/or shift radially with respect to the gas exchanger 320 and/or the bundle of hollow gas exchange fibers 322. In some embodiments, the inflation fluid may be a liquid (e.g., water, saline, etc.). In some embodiments, the inflation fluid may be a gas (e.g., air, oxygen, etc.). Other configurations are also contemplated. In some embodiments, oxygenator 300 may include an inflation lumen (not shown) fluidly coupled to the expandable bladder 390. In some embodiments, the inflation lumen may be fluidly coupled to a source of inflation fluid (not shown). In some embodiments, the source of inflation fluid may be disposed within the oxygenator 300. In some embodiments, the source of inflation fluid may be disposed external to the oxygenator 300 and fluidly coupled thereto, such as with tubing.

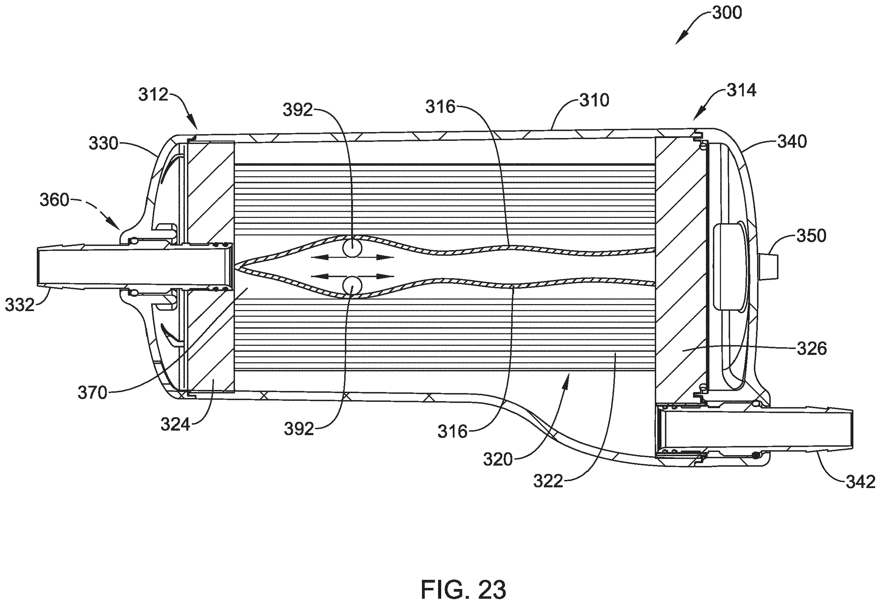

In some embodiments, the oxygenator 300 may comprise an annular ring 392 disposed within the flexible housing 316, as shown in FIG. 23. The annular ring 392 may have an axial length along the central longitudinal axis of the oxygenator 300 and/or the rigid housing 310 less than a length of the flexible housing 316. In some embodiments, the flexible housing 316 may have a first diameter (e.g., a first radial extent) in an unconstrained configuration. In some embodiments, the flexible housing 316 may be under tension between the first potting body 324 and the second potting body 326. In some embodiments, tension applied to the flexible housing 316 may generally define the first diameter of the flexible housing 316. In some embodiments, the annular ring 392 may have an outer diameter greater than the first diameter of the flexible housing 316 such that the annular ring 392 radially expands a portion of the flexible housing 316 against the gas exchanger 320 and/or the bundle of hollow gas exchange fibers 322 to a second diameter greater than the first diameter, as shown in FIG. 23.

In at least some embodiments, the annular ring 392 may be movable axially along and/or within the flexible housing 316. In some embodiments, the annular ring 392 may be movable axially along the flexible housing 316 between the first potting body 324 and the second potting body 326. As the annular ring 392 moves axially along and/or within the flexible housing 316 and/or between the first potting body 324 and the second potting body 326, the annular ring 392 radially expands a portion or a subsection of the flexible housing 316. In some embodiments, expanding a portion or a subsection of the flexible housing 316 may create "waves" (e.g., pressure waves, etc.) within the blood flow within the oxygenator 300 and/or the gas exchanger 320, thereby increasing mixing of blood therein and/or reducing stagnation of blood therein.

The materials that can be used for the various components of the oxygenator and the various elements thereof disclosed herein may include those commonly associated with medical devices. For simplicity purposes, the following discussion refers to the device. However, this is not intended to limit the devices, components, and methods described herein, as the discussion may be applied to other elements, members, components, or devices disclosed herein, such as, but not limited to, the housing, the second cover, the first cover, the diverter, the gas exchanger, the bundle of hollow gas exchange fibers, etc. and/or elements or components thereof.

In some embodiments, the device and/or components thereof may be made from a metal, metal alloy, polymer (some examples of which are disclosed below), a metal-polymer composite, ceramics, combinations thereof, and the like, or other suitable material.

Some examples of suitable polymers may include polytetrafluoroethylene (PTFE), ethylene tetrafluoroethylene (ETFE), fluorinated ethylene propylene (FEP), polyoxymethylene (POM), polyether block ester, polyurethane, polypropylene (PP), polyvinylchloride (PVC), polyether-ester, ether or ester based copolymers (for example, butylene/poly(alkylene ether) phthalate and/or other polyester elastomers), polyamide, elastomeric polyamides, block polyamide/ethers, polyether block amide (PEBA, for example PEBAX^{®}), ethylene vinyl acetate copolymers (EVA), silicones, polyethylene (PE), high-density polyethylene, low-density polyethylene, linear low density polyethylene, polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide, polysulfone, nylon, nylon-12, perfluoro(propyl vinyl ether) (PFA), ethylene vinyl alcohol, polyolefin, polystyrene, epoxy, polyvinylidene chloride (PVdC), poly(styrene-b-isobutylene-b-styrene), polycarbonates, polyisobutylene (PIB), polyisobutylene polyurethane (PIBU), polymethylpentene (PMP), polyurethane silicone copolymers, ionomers, biocompatible polymers, other suitable materials, or mixtures, combinations, copolymers thereof, polymer/metal composites, and the like.

Some examples of suitable metals and metal alloys include stainless steel, such as 304 or 316 stainless steel or variations thereof; mild steel; nickel-titanium alloy such as linear elastic and/or super-elastic nitinol; other nickel alloys such as nickel-chromium-molybdenum alloys, nickel-copper alloys, nickel-cobalt-chromium-molybdenum alloys, nickel-molybdenum alloys, other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys; platinum enriched stainless steel; titanium; combinations thereof; or any other suitable material.

In some embodiments, the device and/or other elements disclosed herein may include and/or be treated with a suitable therapeutic agent. Some examples of suitable therapeutic agents may include anti-thrombogenic agents (such as heparin, heparin derivatives, urokinase, and PPack (dextrophenylalanine proline arginine chloromethyl ketone)); anti-protein and/or anti-bacterial agents (such as 2-methacryroyloxyethyl phosphorylcholine (MPC) and its polymers or copolymers); anti-proliferative agents (such as enoxaparin, angiopeptin, monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-mitotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl ketone, an RGD peptide-containing compound, heparin, anti-thrombin compounds, platelet receptor antagonists, anti-thrombin antibodies, anti-platelet receptor antibodies, aspirin, prostaglandin inhibitors, platelet inhibitors, and tick antiplatelet peptides); vasodilating agents; and agents which interfere with endogenous vasoactive mechanisms.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The disclosure's scope is, of course, defined in the language in which the appended claims are expressed.

## Claims

1. An oxygenator, comprising:
a housing extending from a first end to a second end; and
a bundle of hollow gas exchange fibers extending between the first end and the second end;
wherein at least a portion of the housing is configured to selectively move radially with respect to the bundle of hollow gas exchange fibers.

2. The oxygenator of claim 1, wherein the housing includes a flexible portion disposed between the first end and the second end.

3. The oxygenator of claim 2, wherein the housing further includes a second flexible portion disposed between the first end and the second end, wherein the second flexible portion is configured to selectively move radially with respect to the bundle of hollow gas exchange fibers independently of the flexible portion.

4. The oxygenator of claim 1, wherein a wall of the housing extending between the first end and the second end is configured to shift between radially inward and radially outward positions.

5. The oxygenator of claim 1, wherein the housing includes an actuation apparatus configured to selectively move radially with respect to the bundle of hollow gas exchange fibers.

6. The oxygenator of claim 5, wherein the actuation apparatus is configured to operate in a cyclical manner.

7. The oxygenator of claim 5, wherein the actuation apparatus is configured to operate in a variable manner.

8. The oxygenator of claim 5, wherein the actuation apparatus comprises a plurality of rings disposed between the first end and the second end, wherein the plurality of rings is configured to move radially with respect to the bundle of hollow gas exchange fibers sequentially along the bundle of hollow gas exchange fibers.

9. An oxygenator, comprising:
a rigid housing extending from a first end to a second end;
a flexible housing disposed within the rigid housing; and
a bundle of hollow gas exchange fibers extending between the first end and the second end within the flexible housing;
wherein at least a portion of the flexible housing is configured to selectively move radially with respect to the bundle of hollow gas exchange fibers.

10. The oxygenator of claim 9, wherein the flexible housing is disposed radially outward of the bundle of hollow gas exchange fibers and a fluid is disposed between the rigid housing and the flexible housing, wherein changes in pressure of the fluid cause at least a portion of the flexible housing to move radially with respect to the bundle of hollow gas exchange fibers.

11. The oxygenator of claim 9, wherein the flexible housing is disposed within the bundle of hollow gas exchange fibers and a fluid is disposed within the flexible housing, wherein changes in pressure of the fluid cause at least a portion of the flexible housing to move radially with respect to the bundle of hollow gas exchange fibers.

12. The oxygenator of claim 9, wherein the flexible housing includes an expandable bladder formed within a wall of the flexible housing.

13. The oxygenator of claim 12, wherein the expandable bladder is configured to receive an inflation fluid therein such that changes in pressure or volume of the inflation fluid within the expandable bladder cause at least a portion of the wall of the flexible housing to move radially with respect to the bundle of hollow gas exchange fibers.

14. The oxygenator of claim 12, wherein the expandable bladder is disposed at a position radially outward of the bundle of hollow gas exchange fibers.

15. The oxygenator of claim 9, further comprising an annular ring disposed in contact with the flexible housing, wherein the annular ring is movable axially along the flexible housing.

16. The oxygenator of claim 15, wherein the annular ring is disposed around the bundle of hollow gas exchange fibers between the rigid housing and the flexible housing and has an axial length less than a length of the flexible housing;
wherein the flexible housing has a first diameter in an unconstrained configuration;
wherein the annular ring has an inner diameter less than the first diameter of the flexible housing such that the annular ring radially constricts a portion of the flexible housing disposed therein to a second diameter less than the first diameter.

17. The oxygenator of claim 15, wherein the annular ring is disposed within the flexible housing and has an axial length less than a length of the flexible housing;
wherein the flexible housing has a first diameter in an unconstrained configuration;
wherein the annular ring has an outer diameter greater than the first diameter of the flexible housing such that the annular ring radially expands a portion of the flexible housing to a second diameter greater than the first diameter.

18. An oxygenator, comprising:
a housing extending from a first end to a second end; and
a bundle of hollow gas exchange fibers extending between the first end and the second end;
wherein a radially inward constriction is selectively formable in the housing between the first end and the second end around the bundle of hollow gas exchange fibers;
wherein the radially inward constriction is configured to move axially between the first end and the second end.

19. The oxygenator of claim 18, wherein forming the radially inward constriction causes mixing of blood flowing from a blood inlet port towards a blood outlet port.

20. The oxygenator of claim 19, wherein moving the radially inward constriction axially between the first end and the second end creates a pressure wave within blood flowing from the blood inlet port towards the blood outlet port.
